# EUROPÄISCHE PATENTANMELDUNG

(11) **EP 4 732 871 A1**
(43) Veröffentlichungstag der Anmeldung: **29.04.2026**
(21) Anmeldenummer: 25209665.6
(22) Anmeldetag: 20.10.2025
(51) Int. Cl.: A61M 16/00, A61M 16/18

(54) **ANORDNUNG UND VERFAHREN ZUR KÜNSTLICHEN BEATMUNG EINES PATIENTEN MIT VERRINGERTEM KONDENSIERUNGS-RISIKO**

(30) Priorität: 28.10.2024 DE 102024131337
(71) Anmelder: Drägerwerk AG & Co. KGaA, 23558 Lübeck (DE)
(72) Erfinder: Radomski, Klaus, 23558 Lübeck (DE); Heesch, Ralf, 23558 Lübeck (DE)

(57) **Zusammenfassung**

Die Erfindung betrifft eine Beatmungs-System und ein Beatmungs-Verfahren zur künstlichen Beatmung eines Patienten. Weiterhin betrifft die Erfindung eine Ansteuer-Anordnung und ein Ansteuer-Verfahren zum Ansteuern einer Beatmungs-Anordnung, die zum Beatmungs-System gehört. Ein Narkosemitteldosierer-Bauteil (38) stellt ein Narkotisierungs-Gasgemisch (Ng) mit einer vorgegebenen Soll-Konzentration (con_{req}) eines Narkosemittels (Nm) bereit. Ein Beatmungsgerät (12) fördert ein atembares Gasgemisch mit dem Narkotisierungs-Gasgemisch (Ng) zu einer patientenseitigen Koppeleinheit. Die Soll-Konzentration (con_{req}) wird so vorgegebenen, dass sie höchstens gleich einer berechneten oberen Konzentrations-Schranke (conₘₐₓ) ist. Die obere Konzentrations-Schranke (conₘₐₓ) ist umso größer, je größer die Temperatur im Narkosemitteldosierer-Bauteil (38), im Beatmungsgerät (12) und/oder in einer Umgebung ist. Die Erfindung reduziert das Risiko, dass Narkosemittel (Nm) kondensiert.

## Beschreibung

Die Erfindung betrifft ein Beatmungs-System und ein Beatmungs-Verfahren zur künstlichen Beatmung eines Patienten. Weiterhin betrifft die Erfindung eine Ansteuer-Anordnung und ein Ansteuer-Verfahren zum Ansteuern einer Beatmungs-Anordnung, wobei die angesteuerte Beatmungs-Anordnung ein Bestandteil eines derartigen Beatmungs-Systems ist.

Viele aus dem Stand der Technik bekannte Beatmungs-Anordnungen vermögen einen Patienten künstlich zu beatmen, wobei der Patient narkotisiert oder wenigstens sediert ist. Während der künstlichen Beatmung ist im und / oder am Körper des Patienten eine patientenseitige Koppeleinheit angeordnet. Ein Beatmungsgerät fördert ein atembares Gasgemisch umfassend Sauerstoff und ein Narkotisierungs-Gasgemisch zur patientenseitigen Koppeleinheit. Das Narkotisierungs-Gasgemisch umfasst mindestens ein Narkosemittel. Häufig wird eine gewünschte Konzentration oder auch ein gewünschter Volumenfluss oder Massefluss des Narkosemittels im Narkotisierungs-Gasgemisch vorgegeben. Die tatsächliche Konzentration oder der tatsächliche Volumenfluss oder Massefluss soll dieser Vorgabe möglichst nahekommen, idealerweise mit ihr übereinstimmen.

Der Erfindung liegt die Aufgabe zugrunde, ein Beatmungs-System und ein Beatmungs-Verfahren bereitzustellen, welche ein atembares Gasgemisch mit einem Narkotisierungs-Gasgemisch zu einer patientenseitigen Koppeleinheit fördern und das Narkotisierungs-Gasgemisch mit einer gewünschten Konzentration von Narkosemittel zuverlässiger als bekannte Beatmungs-Systeme und Beatmungs-Verfahren bereitstellen sollen. Weiterhin liegt der Erfindung die Aufgabe zugrunde, eine Ansteuer-Anordnung und ein Ansteuer-Verfahren zum Ansteuern einer Beatmungs-Anordnung bereitzustellen, wobei die Ansteuer-Anordnung und das Ansteuer-Verfahren bewirken sollen, dass das Narkotisierungs-Gasgemisch mit einer gewünschten Konzentration von Narkosemittel zuverlässiger als durch bekannte Ansteuer-Anordnungen und Ansteuer-Verfahren bereitgestellt wird.

Die Aufgabe wird durch ein Beatmungs-System mit den Merkmalen des Anspruchs 1, durch eine Ansteuer-Anordnung mit den Merkmalen des Anspruchs 10, durch ein Ansteuer-Verfahren mit den Merkmalen des Anspruchs 11 und durch ein Beatmungs-Verfahren mit den Merkmalen des Anspruchs 12 gelöst. Vorteilhafte Ausgestaltungen sind in den Unteransprüchen angegeben. Vorteilhafte Ausgestaltungen des erfindungsgemäßen Beatmungs-Systems sind, soweit sinnvoll, auch vorteilhafte Ausgestaltungen der erfindungsgemäßen Ansteuer-Anordnung, des erfindungsgemäßen Ansteuer-Verfahrens und des erfindungsgemäßen Beatmungs-Verfahrens und umgekehrt.

Das erfindungsgemäße Beatmungs-System umfasst eine Beatmungs-Anordnung. Die Beatmungs-Anordnung vermag einen Patienten künstlich zu beatmen. Durch das erfindungsgemäße Beatmungs-Verfahren wird ein Patient künstlich beatmet. Das erfindungsgemäße Beatmungs-Verfahren wird unter Verwendung einer erfindungsgemäßen Beatmungs-Anordnung durchgeführt. Durch die erfindungsgemäße Ansteuer-Anordnung und das erfindungsgemäße Ansteuer-Verfahren wird eine erfindungsgemäße Beatmungs-Anordnung angesteuert. Die Beatmungs-Anordnung und die Ansteuer-Anordnung gehören zum Beatmungs-System. Die Ansteuer-Anordnung kann ein Bestandteil der Beatmungs-Anordnung sein und / oder räumlich von der Beatmungs-Anordnung entfernt sein. Bei der Durchführung des Beatmungs-Verfahren werden die Schritte des Ansteuer-Verfahrens durchgeführt.

Eine patientenseitige Koppeleinheit der Beatmungs-Anordnung lässt sich im und / oder am Körper eines Patienten anordnen, der künstlich beatmet werden soll. Eine Atemmaske auf dem Gesicht eines Patienten und ein Tubus im Körper des Patienten sind zwei Beispiele für eine patientenseitigen Koppeleinheit. Das Ansteuer-Verfahren und das Beatmungs-Verfahren werden durchgeführt, während die patientenseitige Koppeleinheit im und / oder am Körper des Patienten angeordnet ist.

Die Beatmungs-Anordnung umfasst weiterhin ein Narkosemitteldosierer-Bauteil, optional mehrere Narkosemitteldosierer-Bauteile, und ein Beatmungsgerät mit einem Gehäuse. **In** das Gehäuse ist eine Aufnahme eingelassen, optional sind mehrere Aufnahmen eingelassen. Das oder jedes Narkosemitteldosierer-Bauteil ist dauerhaft oder wenigstens zeitweise in die oder jeweils eine Aufnahme im Gehäuse eingesetzt. Bevorzugt lässt sich das Narkosemitteldosierer-Bauteil in die Aufnahme einsetzen und wieder aus dieser Aufnahme entnehmen. **In** die oder jede Aufnahme ist zu jedem Zeitpunkt bevorzugt jeweils genau ein Narkosemitteldosierer-Bauteil eingesetzt, außer in einem Ruhezustand, oder lässt sich einsetzen. Falls die Beatmungs-Anordnung mehrere Narkosemitteldosierer-Bauteile umfasst, ist bevorzugt jedes Narkosemitteldosierer-Bauteil in jeweils eine Aufnahme eingesetzt. Das Ansteuer-Verfahren und das Beatmungs-Verfahren werden durchgeführt, während in die oder mindestens eine Aufnahme das oder ein Narkosemitteldosierer-Bauteil eingesetzt ist.

Das oder jedes Narkosemitteldosierer-Bauteil vermag ein Narkotisierungs-Gasgemisch zu erzeugen und bereitzustellen. Dieses Narkotisierungs-Gasgemisch umfasst mindestens ein Narkosemittel und in einer Ausgestaltung zusätzlich Sauerstoff. Das Narkosemitteldosierer-Bauteil vermag das Narkotisierungs-Gasgemisch dann zu erzeugen, wenn es in die oder eine Aufnahme im Gehäuse eingesetzt ist. Optional sind mehrere Narkosemitteldosierer-Bauteile gleichzeitig in jeweils eine Aufnahme eingesetzt und erzeugen jeweils ein Narkotisierungs-Gasgemisch mit einem Narkosemittel, optional zwei Narkotisierungs-Gasgemische mit zwei verschiedenen Narkosemitteln und / oder mit zwei unterschiedlichen Narkosemittel-Konzentrationen.

Das Beatmungsgerät vermag ein atembares Gasgemisch zu erzeugen. Das atembare Gasgemisch umfasst Sauerstoff und das Narkotisierungs-Gasgemisch, welches das oder mindestens ein eingesetztes Narkosemitteldosierer-Bauteil erzeugt und bereitgestellt hat. Um das atembare Gasgemisch zu erzeugen, verwendet das Beatmungsgerät das Narkotisierungs-Gasgemisch und bevorzugt mindestens ein Gas aus einer Versorgungseinheit, beispielsweise aus einem Versorgungsanschluss oder einem Versorgungsbehälter. Das Beatmungsgerät vermag das atembare Gasgemisch zur patientenseitigen Koppeleinheit zu fördern. Bevorzugt führt das Beatmungsgerät eine Abfolge von Beatmungshüben (ventilation strokes) aus und fördert in jedem Beatmungshub jeweils eine Menge des atembaren Gasgemischs zur patientenseitigen Koppeleinheit. Der Patient kann das atembare Gasgemisch, das zur patientenseitigen Koppeleinheit gefördert ist, einatmen, oder es wird in seinen Körper hinein gefördert.

Ein signalverarbeitendes Steuergerät (control unit) gehört zum erfindungsgemäßen Beatmungs-System, optional zur erfindungsgemäßen Beatmungs-Anordnung, und zur erfindungsgemäßen Ansteuer-Anordnung. Das Steuergerät vermag eine Soll-Konzentration des Narkosemittels im Narkotisierungs-Gasgemisch zu berechnen. Das erfindungsgemäße Ansteuer-Verfahren und das erfindungsgemäße Beatmungs-Verfahren werden unter Verwendung eines solchen Steuergeräts durchgeführt. Die berechnete Soll-Konzentration spezifiziert, mit welcher Konzentration das oder ein Narkosemittel in dem Narkotisierungs-Gasgemisch, welches das Narkosemitteldosierer-Bauteil erzeugt, auftreten soll. Die Soll-Konzentration spezifiziert also eine Vorgabe für das Narkosemitteldosierer-Bauteil. Falls das Narkotisierungs-Gasgemisch mindestens zwei Narkosemittel aufweist, berechnet das Steuergerät bevorzugt für jedes Narkosemittel jeweils eine Soll-Konzentration. Diese Soll-Konzentrationen können sich voneinander unterscheiden.

Das Steuergerät vermag das oder jedes eingesetzte Narkosemitteldosierer-Bauteil anzusteuern. Das Ziel bei dieser Ansteuerung ist, dass das Narkosemitteldosierer-Bauteil das Narkotisierungs-Gasgemisch wie folgt bereitstellt: Das oder jedes Narkosemittel ist im Narkotisierungs-Gasgemisch mit der jeweils berechneten Soll-Konzentration vorhanden. Dieses Ziel lässt sich in der Regel nur annähernd erreichen.

Das Beatmungs-System und die Ansteuer-Anordnung umfassen weiterhin mindestens einen Temperatursensor aus einer Temperatursensor-Gruppe. Das Ansteuer-Verfahren und das Beatmungs-Verfahren werden unter Verwendung mindestens eines solchen Temperatursensors durchgeführt. Die Temperatursensor-Gruppe besteht aus den folgenden drei Temperatursensoren:
- einem Umgebungstemperatur-Sensor,
- einem narkosemitteldosierer-seitigen Temperatursensor und
- einem beatmungsgeräte-seitigen Temperatursensor.

Weil die Temperatursensor-Gruppe aus drei verschiedenen Temperatursensoren besteht und mindestens einer tatsächlich verwendet wird, sind 2^3 - 1 = 7 verschiedene Konfigurationen eines erfindungsgemäßen Beatmungs-Systems und einer erfindungsgemäßen Ansteuer-Anordnung hinsichtlich der Temperatursensoren möglich. Möglich ist, dass die Beatmungs-Anordnung oder die Ansteuer-Anordnung einen weiteren Sensor umfasst, der nicht zur Temperatursensor-Gruppe gehört, beispielsweise einen Druck-Sensor oder einen Volumenfluss-Sensor oder auch einen Temperatursensor, der die Temperatur des atembaren Gasgemischs misst.

Der Umgebungstemperatur-Sensor vermag die Temperatur in einer Umgebung der Beatmungs-Anordnung und damit des Beatmungs-Systems zu messen, und zwar mindestens einmal, bevorzugt mehrmals, sodass ein zeitlicher Verlauf der Umgebungstemperatur gemessen wird. Der Umgebungstemperatur-Sensor oder wenigstens die Messposition des Umgebungstemperatur-Sensors können räumlich von der Beatmungs-Anordnung beabstandet sein.

Möglich ist auch, dass der Umgebungstemperatur-Sensor im Beatmungsgerät angeordnet ist. In einer Realisierungsform misst der Umgebungstemperatur-Sensor im Beatmungsgerät eine Differenz zwischen der Temperatur im Beatmungsgerät und der Temperatur in einer Umgebung des Beatmungsgeräts.

In einer anderen Realisierungsform misst der Umgebungstemperatur-Sensor eine Temperatur im Beatmungsgerät. Eine Abweichung zwischen der Temperatur im Beatmungsgerät und der Umgebungstemperatur ist vorgegeben. Diese Abweichung kann von der Temperatur im Beatmungsgerät und / oder von der bisherigen Nutzungsdauer des Beatmungsgeräts abhängen und wurde bevorzugt vorab empirisch ermittelt. Oft ist die Annahme gerechtfertigt, dass diese Abweichung nicht von der Umgebungstemperatur abhängt. Der Umgebungstemperatur-Sensor kombiniert die gemessene Temperatur im Beatmungsgerät mit der vorgegebenen Abweichung, um die Umgebungstemperatur herzuleiten. Optional wird vorab ein funktionaler Zusammenhang ermittelt, der die Abweichung als Funktion der gemessenen Temperatur im Beatmungsgerät und / oder der Nutzungsdauer des Beatmungsgeräts beschreibt. Dieser funktionale Zusammenhang wird dann auf die gemessene Temperatur im Beatmungsgerät angewendet.

Der narkosemitteldosierer-seitige Temperatursensor vermag mindestens einmal, bevorzugt mehrmals eine Temperatur an einer Narkosemitteldosierer-Messposition zu messen. Die Narkosemitteldosierer-Messposition befindet sich im oder am Narkosemitteldosierer-Bauteil. Bevorzugt steht die Narkosemitteldosierer-Messposition in einem thermischen Kontakt mit einer Oberfläche des Narkosemitteldosierer-Bauteils. Bei eingesetztem Narkosemitteldosierer-Bauteil zeigt diese Oberfläche zur Aufnahme im Gehäuse des Beatmungsgeräts hin. Bevorzugt ist der narkosemitteldosierer-seitige Temperatursensor ein Bestandteil des oder eines Narkosemitteldosierer-Bauteils, er kann aber auch im Inneren des Beatmungsgeräts angeordnet seien. Möglich ist, dass das Beatmungs-System mehrere Narkosemitteldosierer-Bauteile umfasst und jedes dieser Bauteile jeweils einen narkosemitteldosierer-seitigen Temperatursensor umfasst oder wenigstens jeweils eine Narkosemitteldosierer-Messposition bereitstellt.

Der beatmungsgeräte-seitige Temperatursensor vermag eine Temperatur an einer Beatmungsgeräte-Messposition mindestens einmal, bevorzugt mehrmals zu messen. Die Beatmungsgeräte-Messposition befindet sich im oder am Beatmungsgerät, und zwar bevorzugt in der Nähe der oder einer Aufnahme im Gehäuse. Bevorzugt steht die Beatmungsgeräte-Messposition in einem thermischen Kontakt mit einer Oberfläche der Aufnahme im Gehäuse des Beatmungsgeräts. Bei eingesetztem Narkosemitteldosierer-Bauteil zeigt diese Oberfläche zum Narkosemitteldosierer-Bauteil. Bevorzugt ist der beatmungsgeräte-seitige Temperatursensor ein Bestandteil des Beatmungsgeräts. Möglich ist, dass in das Gehäuse mehrere Aufnahmen für jeweils ein Narkosemitteldosierer-Bauteil eingelassen sind und das Beatmungs-System und die Ansteuer-Anordnung für jede Aufnahme jeweils einen beatmungsgeräte-seitigen Temperatursensor umfassen oder wenigstens jeweils eine Beatmungsgeräte-Messposition bereitstellt.

Anmerkung: Die Formulierung wird verwendet, dass ein Sensor eine physikalische Größe zu messen vermag, beispielsweise eine Temperatur an einer Messposition. Diese Formulierung bedeutet, dass der Sensor direkt die physikalische Größe zu messen vermag oder eine andere Größe, die mit der zu messenden Größe korreliert und daher ein Maß für die zu messende physikalische Größe ist. Die Messung liefert mindestens einen Wert der gesuchten physikalischen Größe.

Nachfolgend wird der Begriff "messbare Temperatur" verwendet. Hiermit ist eine Temperatur an einer der drei gerade genannten Messpositionen gemeint. Das Steuergerät ist dazu ausgestaltet, die Soll-Konzentration in Abhängigkeit von mindestens einer messbaren Temperatur zu berechnen. Weil das Beatmungs-System und die Ansteuer-Anordnung mindestens einen Temperatursensor der Temperatursensor-Gruppe umfassen, wird mindestens eine messbare Temperatur tatsächlich gemessen. Eine messbare Temperatur wird entweder von einem tatsächlich vorhandenen Temperatursensor der Temperatursensor-Gruppe gemessen. Oder ein Standardwert für eine messbare Temperatur wird verwendet. Oder mindestens eine messbare Temperatur wird nicht berücksichtigt, um die Soll-Konzentration zu berechnen.

**In** einem Datenspeicher ist ein rechnerauswertbarer funktionaler Zusammenhang abgespeichert. Der Datenspeicher gehört zum Beatmungs-System, optional zur Beatmungs-Anordnung, und zur Ansteuer-Anordnung. Der funktionale Zusammenhang kann auch Bestandteil eines Programms sein, welches das Steuergerät auszuführen vermag. Das Ansteuer-Verfahren und das Beatmungs-Verfahren werden unter Verwendung eines derartigen funktionalen Zusammenhangs durchgeführt.

Der funktionale Zusammenhang spezifiziert eine obere Konzentrations-Schranke für die Konzentration des Narkosemittels im Narkotisierungs-Gasgemisch in Abhängigkeit von mindestens einer messbaren Temperatur, optional von mehreren messbaren Temperaturen. Bei genau einer messbaren Temperatur im funktionalen Zusammenhang führt jeder Wert der auftretenden messbaren Temperatur also zu jeweils einem Wert für die obere Konzentrations-Schranke. Bei mindestens zwei messbaren Temperaturen im funktionalen Zusammenhang führt jede Kombination von Werten der auftretenden messbaren Temperaturen zu jeweils einem Wert für die obere Konzentrations-Schranke. Der funktionale Zusammenhang ist wie folgt ausgestaltet: Wenn die oder eine im funktionalen Zusammenhang auftretende messbare Temperatur ansteigt und bei mehreren messbaren Temperaturen die oder jede andere messbare Temperatur gleichbleibt, wird die obere Konzentrations-Schranke größer oder bleibt mindestens gleich. Mit anderen Worten: Der funktionale Zusammenhang ist in dem oder jedem Argument (eine messbare Temperatur) monoton steigend.

Wie bereits dargelegt, vermag das Steuergerät eine Soll-Konzentration für das oder ein Narkosemittel im Narkotisierungs-Gasgemisch zu berechnen. Das Steuergerät ist dazu ausgestaltet, bei der Berechnung der Soll-Konzentration die folgenden Schritte durchzuführen:
- Das Steuergerät wendet den funktionalen Zusammenhang auf mindestens einen gemessenen Wert der messbaren Temperatur an, bevorzugt auf den zeitlich jüngsten gemessenen Wert, optional auf mehrere Werte. Falls mehrere messbare Temperaturen tatsächlich gemessen werden, wendet das Steuergerät den funktionalen Zusammenhang auf jeweils mindestens einen gemessenen Wert jeder messbaren Temperatur an.
- Durch die Anwendung leitet das Steuergerät einen Wert für die obere Konzentrations-Schranke her.
- Das Steuergerät berechnet die Soll-Konzentration so, dass die Soll-Konzentration höchstens gleich dem hergeleiteten Wert für die obere Konzentrations-Schranke ist. Dadurch hängt die Soll-Konzentration von der gemessenen Temperatur ab.

Möglich ist, dass der funktionale Zusammenhang auf eine bestimmte messbare Temperatur Bezug nimmt, jedoch die Beatmungs-Anordnung für diese Temperatur tatsächlich keinen Temperatursensor umfasst oder dieser Temperatursensor defekt oder ausgeschaltet ist. In diesem Fall verwendet das Steuergerät bevorzugt einen Standardwert, besonders bevorzugt eine vorgegebene untere Schranke oder einen kleinstmöglichen Wert für diese messbare Temperatur. Falls die untere Schranke verwendet wird, "ist man auf der sicheren Seite", was weiter unten beschrieben wird.

Nachfolgend werden mehrere Vorteile der Erfindung beschrieben.

Gewünscht wird, dass ausschließlich gasförmiges Narkosemittel als Teil des Narkotisierungs-Gasgemischs und als Teil des atembaren Gasgemischs von dem oder mindestens einem eingesetzten Narkosemitteldosierer-Bauteil durch eine Fluidführungseinheit zur patientenseitigen Koppeleinheit fließt. In vielen Fällen wird diese gewünschte Situation durch eine ausreichend hohe Umgebungstemperatur und optional durch eine Beheizung dieser Fluidführungseinheit gewährleistet.

Jedes Narkosemittel weist eine Sättigungs-Konzentration (Sattdampf-Konzentration) auf. Falls die Konzentration von Narkosemittel in einem Gasgemisch oberhalb dieser Sättigungs-Konzentration liegt, so kondensiert in der Regel ein Teil des Narkosemittels. In der Regel variiert die Sättigungs-Konzentration von Narkosemittel zu Narkosemittel und hängt außerdem von der Temperatur des Gasgemischs ab. Je größer die Temperatur des Gasgemischs ist, desto größer ist auch die Sättigungs-Konzentration eines bestimmten Narkosemittels. Typischerweise ist die Sättigungs-Konzentration eines Narkosemittels bei einer bestimmten Temperatur eine bekannte Eigenschaft dieses Narkosemittels.

Die unerwünschte Situation kann auftreten, dass gasförmiges Narkosemittel auf der Strecke vom Narkosemitteldosierer-Bauteil zur patientenseitigen Koppeleinheit kondensiert, beispielsweise an einer Innenwand einer verwendeten Fluidführungseinheit. Diese unerwünschte Situation kann insbesondere zu Beginn der künstlichen Beatmung auftreten. Eine Ursache ist die folgende: Das Narkosemitteldosierer-Bauteil wird in einem relativ kühlen Lagerraum vorrätig gehalten und vor Beginn der künstlichen Beatmung in die oder eine Aufnahme eingesetzt. Nachdem das Narkosemitteldosierer-Bauteil in die oder eine Aufnahme eingesetzt ist, verstreicht aufgrund seiner thermischen Masse zwangsläufig eine gewisse Aufwärmphase, bis das Narkosemitteldosierer-Bauteil wenigstens die Umgebungstemperatur erreicht hat. Außerdem kann auch das Beatmungsgerät zu Beginn der künstlichen Beatmung relativ kühl sein. Eine Folge ist: Die Konzentration von Narkosemittel liegt an mindestens einer Stelle in der Beatmungs-Anordnung oberhalb der Sättigungs-Konzentration.

Nachfolgend wird ein Grund dafür beschrieben, dass das Kondensieren von Narkosemittel unerwünscht ist. Das Kondensieren führt nämlich oft häufig zu folgendem unerwünschte Ereignis: Häufig umfasst eine Beatmungs-Anordnung einen Narkosemittel-Sensor. Der Narkosemittel-Sensor vermag die Konzentration eines Narkosemittels in einem Gasgemisch zu messen. Das Gasgemisch ist insbesondere das Narkotisierungs-Gasgemisch oder das atembare Gasgemisch. Die gemessene tatsächliche Narkosemittel-Konzentration wird beispielsweise für eine Regelung (closed-loop control) verwendet. Der Vorgang, dass gasförmiges Narkosemittel kondensiert, beispielsweise auf einer Wand einer Fluidführungseinheit oder auf einer sonstigen Oberfläche der Beatmungs-Anordnung, kann bei dieser Ausgestaltung insbesondere folgende unerwünschte Konsequenz haben: Der Narkosemittel-Sensor misst die Konzentration des Narkosemittels im Narkotisierungs-Gasgemisch und / oder im atembaren Gasgemisch nicht korrekt, sondern misst eine falsche, insbesondere eine zu geringe Narkosemittel-Konzentration. Dies wiederum kann insbesondere bei einer Regelung dazu führen, dass das atembare Gasgemisch eine falsche, insbesondere eine unerwünscht hohe Narkosemittel-Konzentration aufweist.

Die Erfindung reduziert die Gefahr, dass diese unerwünschte Situation auftritt. Diese Wirkung erzielt die Erfindung insbesondere dadurch, dass das Steuergerät einen Wert für die obere Konzentrations-Schranke berechnet und sicherstellt, dass die Soll-Konzentration und bevorzugt auch die tatsächliche Konzentration des Narkosemittels nicht oberhalb dieses berechneten Werts liegt. Dieser berechnete Wert hängt von mindestens einer messbaren Temperatur ab. Je größer die messbare Temperatur ist, desto größer ist auch der berechnete Wert für die obere Konzentrations-Schranke. Bei einer höheren messbaren Temperatur ist nämlich das Risiko geringer als bei einer niedrigen messbaren Temperatur, dass gasförmiges Narkosemittel kondensiert. Jede messbare Temperatur im Sinne der Erfindung beeinflusst nämlich die Temperatur des Narkotisierungs-Gasgemischs oder hängt von der Temperatur des Narkotisierungs-Gasgemischs ab.

Die Erfindung lässt sich in Kombination mit mindestens einer Heizung verwenden, wobei die Heizung das oder ein Narkosemitteldosierer-Bauteil und / oder das Beatmungsgerät beheizt. Die Erfindung erspart aber die Notwendigkeit, ein Segment oder sogar die gesamte Fluidführungseinheit vom Narkosemitteldosierer-Bauteil zur patientenseitigen Koppeleinheit zu heizen, um ein unerwünschtes Kondensieren zu verhindern. Auch in Kombination mit einer Heizung berücksichtigt die Erfindung die Tatsache, dass sowohl ein Narkosemitteldosierer-Bauteil als auch ein Beatmungsgerät jeweils eine thermische Masse aufweisen und daher eine gewisse Zeit verstreicht, bis sie erwärmt sind.

Die Erfindung lässt sich in Verbindung mit einem Temperatursensor verwenden, der die Temperatur des erzeugten Narkotisierungs-Gasgemischs oder des atembaren Gasgemischs misst. Die Erfindung erfordert aber nicht, dass ein solcher Temperatursensor verwendet wird. In manchen Fällen ist es schwieriger, die Temperatur eines Gases zu messen als die Temperatur an den Messpositionen der erfindungsgemäßen Temperatursensoren. Außerdem verhindert ein Temperatursensor allein noch nicht das unerwünschte Kondensieren.

Denkbar wäre auch folgende Ausgestaltung, um ein unerwünschtes Kondensieren zu verhindern: Sichergestellt wird, dass die Soll-Konzentration unter der Sättigungs-Konzentration der niedrigsten möglichen Temperatur liegt, wobei die tatsächliche Temperatur des Narkotisierungs-Gasgemischs oder des atembaren Gasgemischs bei einem Einsatz stets mindestens gleich dieser niedrigsten möglichen Temperatur ist. Diese Ausgestaltung schränkt aber die Einsatzmöglichkeit des Beatmungs-Systems und der Beatmungs-Anordnung ein. Denn dann lässt sich häufig auch bei einer höheren Temperatur des Narkotisierungs-Gasgemischs oder des atembaren Gasgemischs eine höhere Soll-Konzentration nicht erzielen, obwohl bei dieser Temperatur die höhere Soll-Konzentration noch unterhalb der Sättigungs-Konzentration liegt und daher ein Kondensieren nicht zu befürchten ist. Erfindungsgemäß hängt vielmehr die erzielbare Soll-Konzentration von mindestens einer messbaren und tatsächlich gemessenen Temperatur ab, und zwar dergestalt, dass die Soll-Konzentration umso größer ist, je größer die messbare Temperatur ist.

In vielen Fällen lässt die Erfindung sich auf einer bereits bestehenden Beatmungs-Anordnung dadurch realisieren, dass Software auf einem in der Regel ebenfalls bereits vorhandenen Steuergerät der Beatmungs-Anordnung angepasst wird. Die physikalischen Bestandteile, die für die Realisierung der Erfindung benötigt werden, sind nämlich häufig bereits vorhanden. Insbesondere ist oft bereits mindestens ein Temperatursensor der Temperatursensor-Gruppe vorhanden. Die Erfindung erfordert es insbesondere nicht, eine Heizung oder einen Temperatursensor für ein Gasgemisch zu ergänzen.

In einer Ausgestaltung umfassen das Beatmungs-System und die Ansteuer-Anordnung zwei Temperatursensoren aus der Temperatursensor-Gruppe, nämlich den narkosemitteldosierer-seitigen Temperatursensor und den beatmungsgeräte-seitigen Temperatursensor, aber nicht notwendigerweise einen Umgebungstemperatur-Sensor. Beim Ansteuer-Verfahren und beim Beatmungs-Verfahren werden gemäß dieser Ausgestaltung die Temperatur an der Narkosemitteldosierer-Messposition und die Temperatur an der Beatmungsgeräte-Messposition gemessen, aber nicht notwendigerweise die Umgebungstemperatur. Bei dieser Ausgestaltung umfassen das Beatmungs-System und die Ansteuer-Anordnung also nicht notwendigerweise den Umgebungstemperatur-Sensor. Ein Umgebungstemperatur-Sensor kann aber insbesondere dann zusätzlich eingesetzt werden, wenn die Möglichkeit besteht, dass die Umgebungstemperatur zu Beginn eines Einsatzes geringer ist als die Temperatur des Beatmungsgeräts und die Temperatur des oder jedes eingesetzten Narkosemitteldosierer-Bauteils. In dieser Situation steigt häufig die Temperatur des Beatmungsgeräts und die des Narkosemitteldosierer-Bauteils nicht aufgrund einer höheren Umgebungstemperatur an, sondern bleibt gleich oder sinkt sogar.

Gemäß dieser Ausgestaltung spezifiziert bevorzugt der funktionale Zusammenhang die obere Konzentrations-Schranke abhängig von der Temperatur an der Narkosemitteldosierer-Messposition und der Temperatur an der Beatmungsgeräte-Messposition, insgesamt also abhängig von mindestens zwei messbaren Temperaturen.

Das Steuergerät ist gemäß dieser Ausgestaltung dazu ausgestaltet, bei der Herleitung des Werts für die obere Konzentrations-Schranke folgende Schritte durchzuführen: Das Steuergerät wendet den funktionalen Zusammenhang auf den gemessenen Wert der Temperatur an der Narkosemitteldosierer-Messposition und auf den gemessenen Wert der Temperatur an der Beatmungsgeräte-Messposition an.

In vielen Fällen ermöglicht diese Ausgestaltung es, einen größeren Wert für die obere Konzentrations-Schranke herzuleiten, als wenn nur oder stattdessen die gemessene Umgebungstemperatur verwendet worden wäre. Gleichzeitig wird in der Regel das Risiko nicht wesentlich erhöht, dass Narkosemittel kondensiert.

In einer Realisierungsform dieser Ausgestaltung wird eine Aggregierungs-Vorschrift vorgegeben. Die Aggregierungs-Vorschrift spezifiziert eine aggregierte Temperatur als Funktion der Temperatur an der Narkosemitteldosierer-Messposition und der Temperatur an der Beatmungsgeräte-Messposition, optional zusätzlich als Funktion der Umgebungstemperatur. Der funktionale Zusammenhang beschreibt die obere Konzentrations-Schranke als Funktion der aggregierten Temperatur. Die Aggregierungs-Vorschrift ist wie folgt ausgestaltet: Die aggregierte Temperatur ist umso kleiner, je kleiner eine in der Aggregierungs-Vorschrift auftretende messbare Temperatur ist. Die Aggregierungs-Vorschrift ist beispielsweise das Minimum (die kleinere der beiden Temperaturen) oder auch ein gewichtetes Mittel. Der funktionale Zusammenhang spezifiziert dann die obere Konzentrations-Schranke als Funktion der aggregierten Temperatur. Das Steuergerät wendet die Aggregierungs-Vorschrift auf die gemessenen Temperatur-Werte an und dann den funktionalen Zusammenhang auf den resultierenden Wert der aggregierten Temperatur.

Erfindungsgemäß umfassen das Beatmungs-System und die Ansteuer-Anordnung mindestens einen Temperatursensor aus der Temperatursensor-Gruppe. Weiter oben wurde eine Ausgestaltung beschrieben, bei der ein narkosemitteldosierer-seitiger Temperatursensor und ein beatmungsgeräte-seitiger Temperatursensor verwendet werden. Die nachfolgend beschriebene Ausgestaltung lässt sich in Kombination mit diesen beiden Temperatursensoren verwenden, erspart aber die Notwendigkeit, einen solchen Temperatursensor zu verwenden. Vielmehr erfordert die nachfolgend beschriebene Ausgestaltung als Temperatursensor lediglich den Umgebungstemperatur-Sensor. Gemäß dieser Ausgestaltung wird die Umgebungstemperatur gemessen, bevor oder während das Ansteuer-Verfahren und das Beatmungs-Verfahren durchgeführt werden.

Ein Hintergrund ist der folgende: Nach einer Aufwärmphase oder auch einer Abkühlphase weichen die Temperatur des Beatmungsgeräts und die Temperatur des oder jedes eingesetzten Narkosemitteldosierer-Bauteils nur wenig von der Umgebungstemperatur ab. Falls die Soll-Konzentration nach der Aufwärmphase kleiner ist als die Sättigungs-Konzentration bei der Umgebungstemperatur, ist die Gefahr relativ gering, dass Narkosemittel kondensiert. Die Dauer der Aufwärmphase lässt sich in vielen Fällen relativ zuverlässig vorgeben, optional in Abhängigkeit von der Umgebungstemperatur.

Die nachfolgend beschriebene Ausgestaltung gilt für die Situation, dass sich das oder jedes Narkosemitteldosierer-Bauteil in die oder eine Aufnahme einsetzen und wieder aus der Aufnahme entnehmen lässt. Wenn das Narkosemitteldosierer-Bauteil in die Aufnahme eingesetzt ist und die Beatmungs-Anordnung verwendet wird, so gleichen sich sowohl die Temperatur des eingesetzten Narkosemitteldosierer-Bauteils als auch die Temperatur des Beatmungsgeräts an die Umgebungstemperatur an. Nach einer Aufwärmphase oder Abkühlphase stimmen diese drei Temperaturen also annähernd überein. Dies gilt unter der Voraussetzung, dass das Narkotisierungs-Gasgemisch nicht erhitzt wird. Falls das Narkotisierungs-Gasgemisch erhitzt wird, so ist dessen Sättigungs-Konzentration sogar höher.

In einer Ausgestaltung wird ein relativ geringer Standardwert für die obere Konzentrations-Schranke vorgegeben. Dieser Standardwert wird in der Aufwärmphase verwendet. Nach Ablauf der Aufwärmphase wird für die obere Konzentrations-Schranke ein Wert verwendet, der von der gemessenen Umgebungstemperatur abhängt und den das Steuergerät erfindungsgemäß berechnet hat. In der Regel ist der berechnete Wert größer als der vorgegebene Standardwert. Nachfolgend wird eine Realisierungsform beschrieben, wie diese Aufwärmphase detektiert wird.

Gemäß dieser Ausgestaltung werden eine Systemuhr und für jede Aufnahme jeweils ein Eingesetzt-Sensor verwendet. Die Systemuhr kann ein Bestandteil des Steuergeräts sein. Der Eingesetzt-Sensor für eine Aufnahme vermag zu detektieren, ob in diese Aufnahme ein Narkosemitteldosierer-Bauteil eingesetzt ist oder nicht. Die Systemuhr vermag die Zeit zu messen. Das Steuergerät vermag eine Eingesetzt-Zeitdauer zu messen. Diese Eingesetzt-Zeitdauer ist seit dem Einsetzen des Narkosemitteldosierer-Bauteils verstrichen, ohne dass der Narkosemitteldosierer-Bauteil wieder aus der Aufnahme entnommen worden ist. Um die Eingesetzt-Zeitdauer zu messen, verwendet das Steuergerät ein Signal des Eingesetzt-Sensors und ein Signal der Systemuhr.

**In** einer möglichen Fortbildung dieser Ausgestaltung wird in der Aufwärmphase der oben beschriebene Standardwert für die obere Konzentrations-Schranke verwendet. Die nachfolgend beschriebene alternative Fortbildung führt in vielen Fällen zu einem größeren Wert für die obere Konzentrations-Schranke, ohne dass eine erheblich größere Gefahr besteht, dass Narkosemittel kondensiert.

Gemäß dieser alternativen Fortbildung wird ein funktionaler Zusammenhang vorgegeben, der die obere Konzentrations-Schranke in Abhängigkeit von der Umgebungstemperatur und zusätzlich in Abhängigkeit von der Eingesetzt-Zeitdauer spezifiziert. Der funktionale Zusammenhang wird wie folgt vorgegeben: Bei gleichbleibender Eingesetzt-Zeitdauer ist die obere Konzentrations-Schranke umso größer, je größer die Umgebungstemperatur ist. Umgekehrt ist bei gleichbleibender Umgebungstemperatur die obere Konzentrations-Schranke umso größer, je länger die Eingesetzt-Zeitspanne ist. Diese alternative Fortbildung nutzt die folgende Tatsache aus: **In** der Regel ist zu Beginn eines Einsatzes die Umgebungstemperatur größer als die Temperatur des Beatmungsgeräts und die Temperatur des oder jedes Narkosemitteldosierer-Bauteils. Diese Gegebenheit resultiert häufig daher, dass das Beatmungsgerät und das Narkosemitteldosierer-Bauteil in einem relativ kühlen Raum vorrätig gehalten werden, bis sie eingesetzt werden. **In** der Aufwärmphase steigen die Temperatur des Beatmungsgeräts und die Temperatur des oder jedes eingesetzten Narkosemitteldosierer-Bauteils an. Daher steigt die Sättigungs-Konzentration im Laufe der Aufwärmphase an.

Das Steuergerät ist wie folgt ausgestaltet: Um den Wert für die obere Konzentrations-Schranke herzuleiten, wendet das Steuergerät diesen funktionalen Zusammenhang auf den gemessenen Wert der Umgebungstemperatur und zusätzlich auf den gemessenen Wert der Eingesetzt-Zeitdauer an.

Erfindungsgemäß vermag das Steuergerät einen Wert für die obere Konzentrations-Schranke zu berechnen. In einer Ausgestaltung sind das Beatmungs-System und die Ansteuer-Anordnung wie folgt ausgestaltet, und das Ansteuer-Verfahren und das Beatmungs-Verfahren umfasst die folgenden Schritte: Der oder jeder tatsächlich vorhandene Temperatursensor der Temperatursensor-Gruppe misst wiederholt die jeweilige Temperatur, beispielsweise mit jeweils einer vorgegebenen Abtastrate. Das Steuergerät berechnet wiederholt jeweils einen Wert für die obere Konzentrations-Schranke und verwendet hierfür die zeitlich jüngsten gemessenen Temperaturwerte, beispielsweise die N letzten Werte, wobei N eine vorgegebene Anzahl ist, oder die gemessenen Temperaturwerte aus einem gleitende Zeitfenster. In der oben beschriebenen Aufwärmphase wird der berechnete Wert für die obere Konzentrations-Schranke in der Regel immer größer, weil die Umgebungstemperatur größer ist als die der Beatmungs-Anordnung und die des Narkosemittel-Bauteils. Diese Ausgestaltung ermöglicht es, die Narkosemittel-Konzentration im Laufe der Aufwärmphase zu steigern, ohne dass eine große Gefahr besteht, dass Narkosemittel kondensiert.

In einer bevorzugten Ausgestaltung umfasst die Beatmungs-Anordnung einen Narkosemittel-Sensor. Der Narkosemittel-Sensor vermag die Konzentration des Narkosemittels im erzeugten Narkotisierungs-Gasgemisch zu messen. Der Narkosemittel-Sensor befindet sich bevorzugt zwischen der oder jeder Aufnahme einerseits und der patientenseitigen Koppeleinheit andererseits und ist bevorzugt im Inneren des Gehäuses des Beatmungsgeräts angeordnet. Bevorzugt führt das Steuergerät eine Regelung (closed-loop control) durch. Das Ziel bei dieser Regelung ist, dass die gemessene tatsächliche Konzentration des Narkosemittels im Narkotisierungs-Gasgemisch oder im atembaren Gasgemisch gleich der berechneten Soll-Konzentration ist. Für diese Regelung empfängt und verarbeitet das Steuergerät ein Signal des Narkosemittel-Sensors. Bei einer großen Regelabweichung, also einer großen Abweichung zwischen der tatsächlichen Konzentration und der Soll-Konzentration, steuert das Steuergerät das Narkosemitteldosierer-Bauteil mit dem Ziel an, die Regelabweichung zu verringern.

Die Erfindung reduziert das Risiko, dass Narkosemittel innerhalb des Narkosemittel-Sensors kondensiert. Kondensiertes Narkosemittel kann zu einem falschen Messergebnis des Narkosemittel-Sensors und manchmal zu einer Beschädigung einer Komponente des Narkosemittel-Sensors führen.

Der beatmungsgeräte-seitige Temperatursensor vermag die Temperatur an der Beatmungsgeräte-Messposition zu messen. In einer Ausgestaltung steht die Beatmungsgeräte-Messposition in einem thermischen Kontakt mit dem Narkosemittel-Sensor. Diese Realisierungsform verringert noch weiter das Risiko, dass Narkosemittel im Narkosemittel-Sensor kondensiert.

Bei der bislang beschriebenen Ausgestaltung wurde der Begriff "das Narkosemittel" verwendet. Möglich ist, dass unterschiedliche Narkosemittel verwendet werden. Insbesondere ist häufig mindestens eine der folgenden Anwendungen möglich:
- Eine erfindungsgemäße Beatmungs-Anordnung vermag einen Patienten mit einem Narkotisierungs-Gasgemisch zu versorgen, welches zwei verschiedene Narkosemittel enthält. Diese beiden Narkosemittel stammen in der Regel von zwei verschiedenen Narkosemitteldosierer-Bauteilen, die gleichzeitig oder wenigstens zeitlich überlappend in zwei verschiedene Aufnahmen im Gehäuse des Beatmungsgeräts eingesetzt sind.
- Oder dieselbe erfindungsgemäße Beatmungs-Anordnung vermag einen ersten Patienten mit einem ersten Narkotisierungs-Gasgemisch und anschließend einen zweiten Patienten mit einem zweiten Narkotisierungs-Gasgemisch zu versorgen, wobei das erste Narkotisierungs-Gasgemisch ein erstes Narkosemittel und das zweite Narkotisierungs-Gasgemisch ein zweites Narkosemittel, das vom ersten Narkosemittel verschieden ist, umfasst.
- Beispielsweise wird in dieselbe Aufnahme zunächst ein erstes Narkosemitteldosierer-Bauteil mit dem ersten Narkosemittel und anschließend ein zweites Narkosemitteldosierer-Bauteil mit dem zweiten Narkosemittel eingesetzt und verwendet.
- Oder eine erste erfindungsgemäße Beatmungs-Anordnung beatmet einen Patienten mit einem ersten Narkotisierungs-Gasgemisch, und eine zweite erfindungsgemäße Beatmungs-Anordnung beatmet denselben oder einen anderen Patienten mit einem zweiten Narkotisierungs-Gasgemisch, wobei diese beiden Narkotisierungs-Gasgemische wiederum unterschiedliche Narkosemittel und / oder unterschiedliche Narkosemittel-Konzentrationen umfassen.

Wie bereits dargelegt, hängt die Sättigungs-Konzentration eines Narkosemittels von der Temperatur eines Gasgemischs, welches das Narkosemittel umfasst, ab und unterscheidet sich bei gleicher Temperatur außerdem von Narkosemittel zu Narkosemittel. In einer Ausgestaltung wird der funktionale Zusammenhang für die obere Konzentrations-Schranke so vorgegeben, dass seine erfindungsgemäße Anwendung für jedes in Betracht kommende Narkosemittel mit hoher Sicherheit verhindert, dass Narkosemittel kondensiert. Beispielsweise wird das Narkosemittel mit der geringsten Sättigungs-Konzentration verwendet, um den funktionalen Zusammenhang aufzustellen. Die nachfolgend beschriebene alternative Ausgestaltung unterscheidet zwischen verschiedenen Narkosemitteln und ermöglicht daher in vielen Fällen, für mindestens ein Narkosemittel eine höhere Soll-Konzentration zu verwenden als für ein anderes, was die Einsatzmöglichkeit der Beatmungs-Anordnung vergrößert, verglichen mit der Verwendung der geringsten Sättigungs-Konzentration.

Gemäß dieser alternativen Ausgestaltung wird eine Menge mit mindestens zwei in Betracht kommenden Narkosemitteln vorgegeben, also eine Auflistung mit diesen Narkosemitteln. Für jedes Narkosemittel der Narkosemittel-Menge wird in rechnerauswertbarer Form jeweils ein funktionaler Einzel-Zusammenhang vorgegeben. Dieser funktionale Einzel-Zusammenhang gilt für dieses Narkosemittel und spezifiziert die obere Konzentrations-Schranke abhängig von der oder jeder messbaren Temperatur. Mit steigender messbarer Temperatur wird die im funktionalen Einzel-Zusammenhang spezifizierte obere Konzentrations-Schranke größer oder bleibt mindestens gleich, so wie dies weiter oben für den einen funktionalen Zusammenhang dargelegt wurde. Die Einzel-Zusammenhänge können sich von Narkosemittel zu Narkosemittel voneinander unterscheiden.

Gemäß der alternativen Ausgestaltung vermag das Steuergerät für jedes in Betracht kommende Narkosemittel jeweils eine Soll-Konzentration zu berechnen. Hierfür erfasst das Steuergerät eine Vorgabe oder eine Messung, welches Narkosemittel in dem Narkotisierungs-Gasgemisch enthalten ist, welches das Narkosemitteldosierer-Bauteil erzeugen soll, und wendet den funktionalen Einzel-Zusammenhang für dieses Narkosemittel auf den jeweiligen gemessenen Wert der oder jeder messbaren Temperatur an. Die Anwendung liefert einen Wert für die obere Konzentrations-Schranke, der unter der Sättigungsgrenze dieses Narkosemittels liegt.

Diese Ausgestaltung lässt sich in Kombination mit einer Anwendung realisieren, bei der die Beatmungs-Anordnung tatsächlich nur ein einziges Narkosemittel verwendet. Bevorzugt ist dann vorgegeben oder wird gemessen, welches Narkosemittel dies ist, und das Steuergerät wendet immer den funktionalen Einzelzusammenhang für dieses eine vorgegebene Narkosemittel an. Weil aber mehrere Einzel-Zusammenhänge vorgegeben und abgespeichert sind, lässt sich eine erfindungsgemäße Beatmungs-Anordnung später doch für ein anderes Narkosemittel verwenden. Die steigert die Flexibilität.

In einer bevorzugten Fortbildung der Ausgestaltung mit den mehreren vorgegebenen Einzel-Zusammenhängen vermag das Steuergerät für die oder jede Aufnahme im Gehäuse zu erfassen, welches Narkosemittel das Narkotisierungs-Gasgemisch umfasst, welches von einem Narkosemitteldosierer-Bauteil in dieser Aufnahme erzeugt und bereitgestellt wird. Beispielsweise erfasst das Steuergerät eine Eingabe eines Benutzers. Oder die Beatmungs-Anordnung umfasst für jede Aufnahme jeweils ein Lesegerät, wobei das Lesegerät eine Kennzeichnung auf einer Oberfläche oder in einem Datenspeicher des in diese Aufnahme eingesetzten Narkosemitteldosierer-Bauteils zu lesen vermag. Die Kennzeichnung kann beispielsweise auf einem NFC-Chip, insbesondere RFID-Chip, abgespeichert sein oder ein Strichmuster oder einen QR-Code oder eine Abfolge von alphanumerischen Zeichen oder eine Farbcodierung umfassen. Das Steuergerät wertet ein Signal des Lesegeräts aus und "weiß" daher, welches Narkosemittel das Narkotisierungs-Gasgemisch vom Narkosemitteldosierer-Bauteil in dieser Aufnahme umfasst. Das Steuergerät wählt den funktionalen Einzel-Zusammenhang für das erfasste Narkosemittel aus und wendet diesen auf den jeweils gemessenen Wert jeder messbaren Temperatur an.

Erfindungsgemäß vermag das Steuergerät eine Soll-Konzentration für die Konzentration des Narkosemittels im Narkotisierungs-Gasgemisch zu berechnen. In einer Ausgestaltung vermag das Steuergerät eine Vorgabe zu erfassen. Die erfasste Vorgabe spezifiziert eine Konzentration oder einen Volumenfluss oder einen Massefluss des Narkosemittels in dem zu erzeugenden Narkotisierungs-Gasgemisch. Die Vorgabe kann von einem Benutzer oder von einer übergeordneten Steuerung stammen. Insbesondere kann die Vorgabe spezifizieren, welche Konzentration das Narkosemittel im atembaren Gasgemisch, das die patientenseitigen Koppeleinheit erreicht, aufweisen soll. Das Steuergerät vermag die Soll-Konzentration abhängig von der erfassten Vorgabe herzuleiten und dadurch zu berechnen. Im einfachsten Fall verwendet das Steuergerät die erfasste Vorgabe als die Soll-Konzentration. Gemäß dieser Ausgestaltung umfassen das Ansteuer-Verfahren und das Beatmungs-Verfahren die entsprechenden Schritte.

In einer Ausgestaltung umfassen das Beatmungs-System und die Ansteuer-Anordnung eine Eingabeeinheit. Mithilfe dieser Eingabeeinheit vermag ein Benutzer die gerade beschriebene Vorgabe für eine Konzentration oder einen Volumenfluss oder einen Massefluss des Narkosemittels zu tätigen, und die Eingabeeinheit erfasst die Benutzereingabe. Bevorzugt vermag die Eingabeeinheit einem Benutzer einen Wertebereich anzubieten. Der Benutzer kann die Vorgabe dadurch tätigen, dass er einen Wert aus dem angebotenen Wertebereich auswählt, beispielsweise mit einem Schieberegler. Die Eingabeeinheit vermag diese Auswahl zu erfassen, und die erfasste Auswahl wird an das Steuergerät übermittelt.

Bevorzugt vermag das Steuergerät vorab eine obere Grenze für diesen Wertebereich zu berechnen. Um diese obere Schranke zu berechnen, verwendet das Steuergerät den erfindungsgemäß hergeleiteten Wert für die obere Konzentrations-Schranke. Das Steuergerät berechnet die obere Grenze für den Wertebereich wie folgt: Jeder Wert aus dem Wertebereich führt zu einer Soll-Konzentration des Narkosemittels im Narkotisierungs-Gasgemisch, die höchstens gleich dem Wert für die obere Konzentrations-Schranke ist. Dadurch wird folgendes unerwünschte Ereignis verhindert: Der Benutzer wählt aus dem Wertebereich einen Wert aus, aber dieser Wert würde zu einer zu hohen Narkosemittel-Konzentration führen, also zu einer Narkosemittel-Konzentration, bei der eine relativ große Gefahr des Narkosemittel-Kondensierens besteht. Die Erfindung erspart insbesondere die Notwendigkeit, von einer Benutzereingabe abzuweichen oder eine Meldung an den Benutzer auszugeben, der zufolge die Benutzereingabe nicht realisiert werden kann.

Bevorzugt stellt die Beatmungs-Anordnung einen Beatmungskreislauf bereit. Das Gasgemisch, welches der Patient ausgeatmet hat, fließt von der patientenseitigen Koppeleinheit zurück zum Beatmungsgerät. Weil das atembare Gasgemisch mindestens ein Narkosemittel aufweist, umfasst in der Regel auch das ausgeatmete Gasgemisch dieses Narkosemittel. Weil ein Beatmungskreislauf bereitgestellt ist, wird die Gefahr verringert, dass dieses Narkosemittel in eine Umgebung der Beatmungs-Anordnung gelangt.

Im Folgenden wird die Erfindung anhand eines Ausführungsbeispiels beschrieben. Hierbei zeigt
- Figur 1: schematisch die Beatmungs-Anordnung des Ausführungsbeispiels;
- Figur 2: einen Ausschnitt aus der Beatmungs-Anordnung von Figur 1;
- Figur 3: einen Ausschnitt aus einer Beatmungs-Anordnung mit zwei Narkosemitteldosierer-Bauteilen;
- Figur 4: schematisch, wie das Steuergerät die obere Konzentrations-Schranke dann berechnet, wenn drei Temperatursensoren vorhanden sind;
- Figur 5: schematisch, wie das Steuergerät die obere Konzentrations-Schranke dann berechnet, wenn nur ein Sensor für die Umgebungstemperatur vorhanden ist.

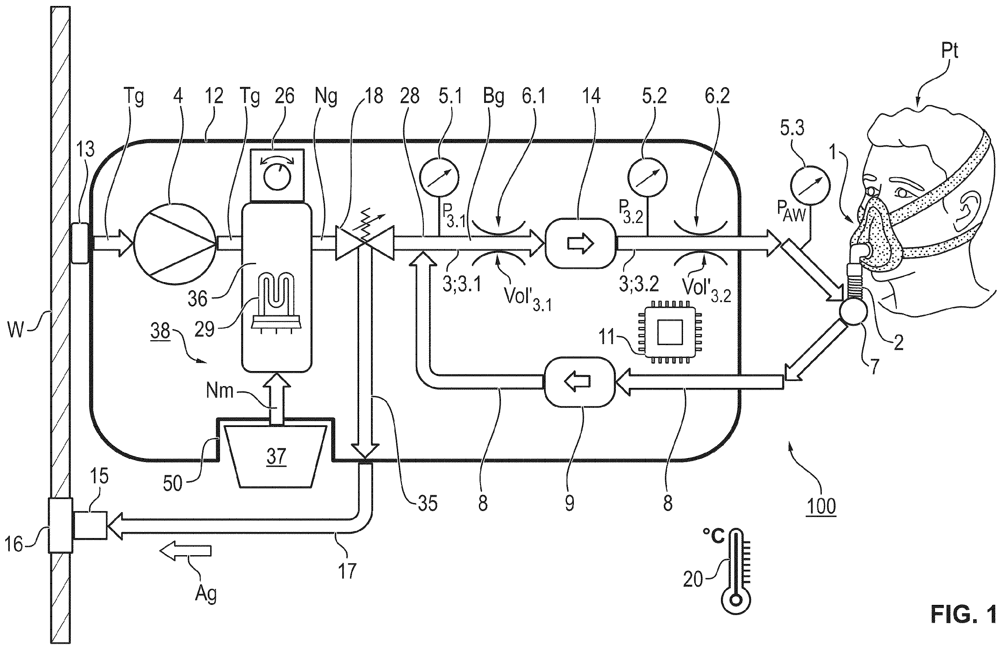

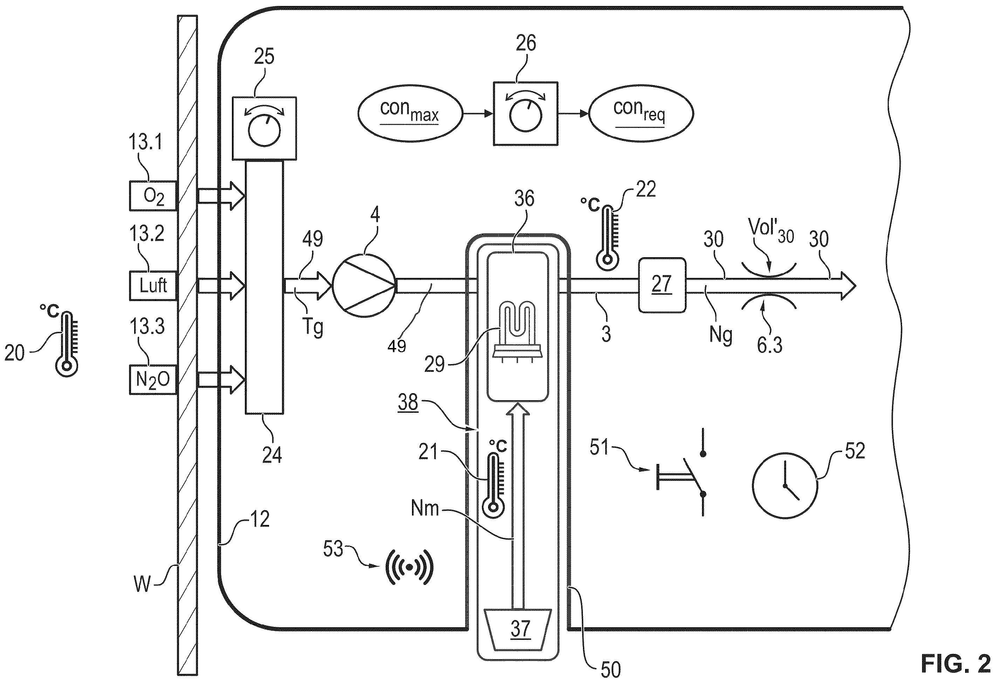

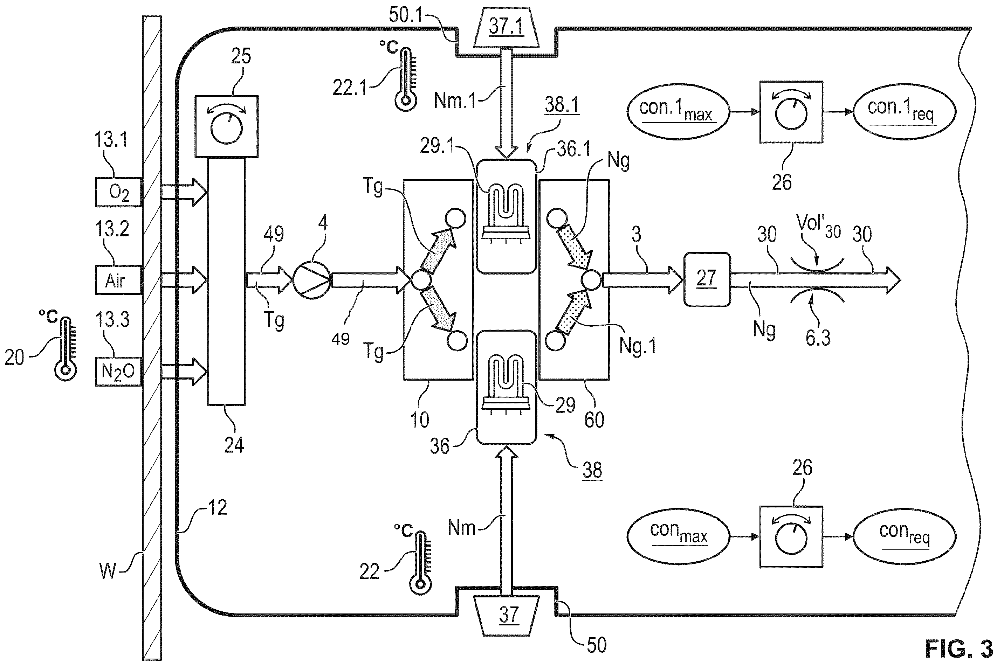

Figur 1 zeigt schematisch eine bevorzugte Anwendung der Erfindung in einer Beatmungs-Anordnung, die einen Beatmungskreislauf bereitstellt. Figur 2 zeigt schematisch einen Ausschnitt der Beatmungs-Anordnung von Figur 1. Figur 3 zeigt schematisch einen Ausschnitt einer Beatmungs-Anordnung mit zwei Narkosemitteldosierer-Bauteilen. Gleiche Bezugszeichen haben die gleichen Bedeutungen. In diesen drei Figuren sind außerdem Bestandteile einer Ansteuer-Anordnung gezeigt. Das Beatmungs-System des Ausführungsbeispiels umfasst die nachfolgend beschriebene Beatmungs-Anordnung und die teilweise gezeigte und weiter unten beschriebene Ansteuer-Anordnung.

Eine Beatmungs-Anordnung beatmet künstlich einen Patienten Pt. Am und / oder im Körper des Patienten Pt ist eine patientenseitige Koppeleinheit angebracht, im Ausführungsbeispiel eine Atemmaske 1 auf seinem Gesicht, optional ein Tubus im Körper des Patienten Pt. Eine Inspirations-Fluidführungseinheit, beispielsweise ein Schlauch, umfasst zwei nachfolgend beschriebene Segmente 3.1 und 3.2 und verbindet ein schematisch gezeigtes Beatmungsgerät 100 mit den beiden Schenkeln eines Y-Stücks 7. Die patientenseitige Koppeleinheit 1 steht über eine patientenseitige Fluidführungseinheit 2 in einer Fluidverbindung mit dem Basisteil des Y-Stücks 7.

Im Ausführungsbeispiel ist der Patient Pt mit Hilfe eines Narkosemittels narkotisiert oder wenigstens sediert. Das Beatmungsgerät 100 umfasst ein Gehäuse 12 und ist mit einer schematisch gezeigten Versorgungsanschluss-Anordnung 13 für Atemluft und Sauerstoff und optional für Druckluft und / oder für mindestens einen Bestandteil eines Trägergases für Narkosemittel verbunden. Die Versorgungsanschluss-Anordnung 13 ist in eine Wand W oder in eine Versorgungseinheit, beispielsweise in eine Deckenversorgungseinheit, eingelassen. Ein Teil der Inspirations-Fluidführungseinheit befindet sich innerhalb des Gehäuses 12, der übrige Teil außerhalb des Gehäuses 12.

Im Ausführungsbeispiel ist in die Wand W die Versorgungsanschluss-Anordnung 13 eingelassen. Die Versorgungsanschluss-Anordnung 13 umfasst in diesem Beispiel drei einzelne Versorgungsanschlüsse, nämlich einen Versorgungsanschluss 13.1 für reinen Sauerstoff (O₂), einen Versorgungsanschluss 13.2 für Atemluft (Air) und einen Versorgungsanschluss 13.3 für Lachgas (N₂O), vgl. Figur 2 und Figur 3. Die drei Gase aus den drei Versorgungsanschlüssen 13.1, 13.2, 13.3 werden einem ansteuerbaren Trägergas-Mischer 24 zugeführt. Mithilfe einer Bedieneinheit 25 kann ein Benutzer festlegen, in welchem Mischungsverhältnis die drei Gase Sauerstoff, Atemluft und Lachgas zum Trägergas vermischt werden sollen.

Der Trägergas-Mischer 24 erzeugt entsprechend der Ansteuerung ein Trägergas Tg, welches mindestens eines der drei möglichen Bestandteile reinen Sauerstoff, Atemluft und Lachgas aufweist. Eine Trägergas-Fluidführungseinheit 49 leitet das Trägergas Tg zu einem Narkosemitteldosierer 36. Der Narkosemitteldosierer 36 erhält einerseits das Trägergas Tg vom Trägergas-Mischer 24 und andererseits ein flüssiges Narkosemittel Nm aus einem Narkosemittelbehälter 37.

In einer Realisierungsform ist der Narkosemittelbehälter 37 in eine Aufnahme 50 im Gehäuse 12 eingesetzt, vgl. Figur 1 und Figur 3. Der Narkosemittelbehälter 37 lässt sich im Ausführungsbeispiel aus der Aufnahme 50 entnehmen und wieder in die Aufnahme 50 einsetzen. Dadurch lässt sich beispielsweise ein verbrauchter Narkosemittelbehälter 37 durch einen neuen Narkosemittelbehälter ersetzen, wobei sich im neuen Narkosemittelbehälter dasselbe oder ein anderes Narkosemittel befinden kann.

In einer anderen Realisierungsform gehören der Narkosemitteldosierer 36 und der Narkosemittelbehälter 37 zu einem Narkosemitteldosierer-Bauteil 38 (Vapor-Bauteil). Das Narkosemitteldosierer-Bauteil 38 lässt sich als Ganzes aus der Aufnahme 50 entnehmen, und dasselbe oder ein anderes Narkosemitteldosierer-Bauteil 38 lässt sich wieder in die Aufnahme 50 einsetzen, vgl. Figur 2. Das Narkosemitteldosierer-Bauteil 38 umfasst mindestens zwei pneumatische Koppelstellen. In die Aufnahme 50 sind zwei korrespondierende Koppelstellen eingelassen. Bei eingesetztem Narkosemitteldosierer-Bauteil 38 fließt einerseits das Trägergas Tg aus dem Beatmungsgerät 100 in das Narkosemitteldosierer-Bauteil 38. Andererseits fließt ein Narkotisierungs-Gasgemisch Ng aus dem Narkosemitteldosierer-Bauteil 38 in das Beatmungsgerät 100. Bevorzugt versorgt das Beatmungsgerät 100 außerdem das Narkosemitteldosierer-Bauteil 38 mittels zweier korrespondierender elektrischer Schnittstellen mit elektrischer Energie.

Nachfolgend wird abkürzend von "dem Narkosemitteldosierer-Bauteil 38" gesprochen, und damit können sowohl die Realisierungsformen gemäß Figur 1 und Figur 3 als auch die Realisierungsform gemäß Figur 2 gemeint sein.

Ein Kontaktschalter 51 detektiert, ob in die Aufnahme 50 ein Narkosemitteldosierer-Bauteil 38 eingesetzt ist oder nicht. Die in Figur 2 gezeigte Position des Kontaktschalters 51 ist nur beispielhaft zu verstehen. Eine Systemuhr 52 misst die Zeitspanne, die verstrichen ist, seitdem ein Narkosemitteldosierer-Bauteil 38 in die Aufnahme 50 eingesetzt und noch nicht wieder entnommen worden ist. Ein Lesegerät 53 liest eine Kennzeichnung auf einem Narkosemitteldosierer-Bauteil 38, das in die Aufnahme 50 eingesetzt ist, und zwar bevorzugt berührungslos. Diese Kennzeichnung spezifiziert, welches Narkosemittel das Narkotisierungs-Gasgemisch Ng enthält, das vom eingesetzten Narkosemitteldosierer-Bauteil 38 erzeugt und bereitgestellt wird.

Bei der Ausgestaltung gemäß Figur 3 sind in das Gehäuse 12 zwei Aufnahmen 50, 50.1 für jeweils einen Narkosemittelbehälter 37, 37.1 eingelassen. Der Narkosemittelbehälter 37 gehört zum Narkosemitteldosierer-Bauteil 38, der weitere Narkosemittelbehälter 37.1 zu einem weiteren Narkosemitteldosierer-Bauteil 38.1. Das Narkosemitteldosierer-Bauteil 38 umfasst weiterhin den Narkosemitteldosierer 36, das weitere Narkosemitteldosierer-Bauteil 38.1 einen weiteren Narkosemitteldosierer 36.1. Der weitere Narkosemittelbehälter 37.1 enthält ein weiteres Narkosemittel Nm.1. Dieses kann das gleiche sein wie das Narkosemittel Nm im Narkosemittelbehälter 37 oder auch ein anderes Narkosemittel. An der weiteren Aufnahme 50.1 sind ein weiterer Kontaktschalter und ein weiteres Lesegerät angeordnet (nicht gezeigt), wobei der weitere Kontaktschalter das Ereignis detektiert, dass ein weiteres Narkosemitteldosierer-Bauteil 38.1 in die Aufnahme 50.1 eingesetzt ist, und wobei das weitere Lesegerät eine Kennzeichnung für das Narkosemittel Nm.1 des eingesetzten Narkosemitteldosierer-Bauteils 38.1 liest. Die Systemuhr 52 vermag auch zu messen, welche Zeitspanne verstrichen ist, seit in die weitere Aufnahme 50.1 ein weiteres Narkosemitteldosierer-Bauteil 38.1 eingesetzt worden ist.

Der Narkosemitteldosierer 36, 36.1 verdunstet oder verdampft in einer Einspeisekammer (nicht gezeigt) flüssiges Narkosemittel aus dem Narkosemittelbehälter 37, 37.1 und speist mindestens ein gasförmiges Narkosemittel Nm, Nm.1 in einen Strom des Trägergases Tg ein. Schematisch wird eine Heizung 29 in der Einspeisekammer des Narkosemitteldosierers 36 gezeigt. In der Einspeisekammer des weiteren Narkosemitteldosierers 36.1 ist eine weitere Heizung 29.1 angeordnet. Diese Heizung 29, 29.1 trägt dazu bei, dass ein flüssiges Narkosemittel Nm, Nm.1, das in die Einspeisekammer eingespeist wird, verdampft oder verdunstet wird. In einer alternativen Realisierungsform spritzt der Narkosemitteldosierer 36, 36.1 das flüssige Narkosemittel Nm, Nm.1 in die Einspeisekammer ein, und das eingespritzte Narkosemittel Nm, Nm.1 wird verdampft oder verdunstet, sodass ein Sattdampf mit einer Narkosemittel-Konzentration nahe der Sättigungs-Konzentration entsteht. Dieser Sattdampf wird mit dem Trägergas-Strom vermischt. Das Einspritzen kann kontinuierlich oder gepulst durchgeführt werden.

Durch das Einspeisen erzeugt der Narkosemitteldosierer 36 ein Gemisch aus dem Trägergas Tg und dem Narkosemittel Nm aus dem Narkosemittelbehälter 37. Entsprechend erzeugt der weitere Narkosemitteldosierer 36.1 ein Gemisch aus dem gleichen Trägergas Tg und dem gleichen oder einem anderen Narkosemittel Nm.1 aus dem Narkosemittelbehälter 37.1. Dieses Gemisch wird nachfolgend als Narkotisierungs-Gasgemisch Ng bzw. Ng.1 bezeichnet. Das Narkotisierungs-Gasgemisch Ng.1 kann Narkosemittel mit einer anderen Konzentration und / oder ein anderes Narkosemittel enthalten als das Narkotisierungs-Gasgemisch Ng.

Im Ausführungsbeispiel kann ein Benutzer mithilfe einer Bedieneinheit 26 eine Soll-Konzentration con_{req} des Narkosemittels Nm im Narkotisierungs-Gasgemisch Ng vorgeben. Hierbei wählt der Benutzer einen Wert aus einem vorgegebenen Wertebereich vor. Eine obere Konzentrations-Schranke conₘₐₓ für die Soll-Konzentration con_{req} begrenzt diesem Wertebereich nach oben. Der Benutzer kann also als Soll-Konzentration con_{req} höchstens den Wert conₘₐₓ für die obere Konzentrations-Schranke Conₘₐₓ vorgeben. Das Entsprechende gilt für die Soll-Konzentration con.1_{req} des weiteren Narkosemittels Nm.1 im Narkotisierungs-Gasgemisch Ng.1 vom weiteren Narkosemitteldosierer-Bauteil 38.1: Die Soll-Konzentration con.1_{req} ist höchstens genauso wie ein berechneter Wert con.1ₘₐₓ. Die beiden Soll-Konzentrationen con_{req} und con.1_{req} lassen sich bevorzugt unabhängig voneinander festlegen.

In einer alternativen, nicht gezeigten Ausgestaltung kann ein Benutzer mithilfe einer entsprechenden Bedieneinheit (nicht gezeigt) eine Soll-Konzentration des Narkosemittels Nm im Beatmungs-Gasgemisch Bg vorgeben, bevorzugt als Soll-Konzentration am Y-Stück 7 und damit in der patientenseitigen Koppeleinheit 1. Das Steuergerät 11 leitet aus dieser Vorgabe und aus weiteren Vorgaben und / oder Messwerten die Soll-Konzentration con_{req} des Narkosemittels Nm im Narkotisierungs-Gasgemisch Ng her. Wiederum ist die Soll-Konzentration con_{req} höchstens so groß wie die obere Konzentrations-Schranke conₘₐₓ.

In Figur 2 wird schematisch gezeigt, dass aus einer Benutzervorgabe mittels der Bedieneinheit 26 eine Soll-Konzentration con_{req} resultiert. In Figur 3 wird außerdem schematisch gezeigt, dass aus einer Benutzervorgabe eine Soll-Konzentration con.1_{req} resultiert. Ein signalverarbeitendes Steuergerät (control unit) 11 verwendet diese Soll-Konzentration con_{req}, um das eingesetzte Narkosemitteldosierer-Bauteil 38 anzusteuern. Das Ziel bei dieser Ansteuerung ist, dass die tatsächliche Konzentration des Narkosemittels Nm im Narkotisierungs-Gasgemisch Ng gleich der Soll-Konzentration con_{req} ist. Entsprechend resultiert aus einer Benutzervorgabe eine Soll-Konzentration con.1_{req} für das Narkosemittel Nm.1 im weiteren Narkotisierungs-Gasgemisch Ng.1. Bei der Ansteuerung wird beispielsweise ein Volumenfluss oder Massefluss des Trägergases Tg und / oder die Wärmeenergie, die die Heizung 29, 29.1 des Narkosemitteldosierer-Bauteils 38, 38.1 abgibt, verändert. Oder ein Volumenfluss oder Massenfluss oder eine Pulsrate, mit der Narkosemittel in die Einspeisekammer eingespritzt wird, wird verändert.

Bei der Ausgestaltung gemäß Figur 3 sind gleichzeitig zwei Narkosemitteldosierer-Bauteile 38 und 38.1 in die beiden Aufnahmen 50 und 50.1 eingesetzt. Bevorzugt wählt der Benutzer für jedes Narkosemitteldosierer-Bauteil 38, 38.1 jeweils eine Soll-Konzentration aus einem Wertebereich aus, insgesamt also zwei Soll-Konzentrationen con_{req}, con.1_{req} aus zwei Wertebereichen. Jede Wertebereich ist nach oben durch jeweils einen Wert conₘₐₓ, con.1ₘₐₓ für eine obere Konzentrations-Schranke Conₘₐₓ, Con.1ₘₐₓ begrenzt. Diese beiden Werte können sich voneinander unterscheiden.

In der Ausgestaltung gemäß Figur 3 leitet ein pneumatisches Schaltventil 10 das Trägergas Tg je nach Stellung des Schaltventils 10 wahlweise zum Narkosemitteldosierer-Bauteil 38 und damit zum Narkosemitteldosierer 36 oder zum weiteren Narkosemitteldosierer-Bauteil 38.1 und damit zum weiteren Narkosemitteldosierer 36.1. Eine Zusammenführeinheit 60 leitet sowohl das Narkotisierungs-Gasgemisch Ng aus dem Narkosemitteldosierer-Bauteil 38 als auch das weitere Narkotisierungs-Gasgemisch Ng.1 aus dem weiteren Narkosemitteldosierer-Bauteil 38.1 in eine Zuführ-Fluidführungseinheit 30.

In einer alternativen Ausgestaltung wird das Trägergas Tg mindestens zeitweise zu beiden Narkosemitteldosierer-Bauteilen 38, 38.1 und damit zu beiden eingesetzten Narkosemitteldosierern 36, 36.1 geleitet. Gemäß dieser alternativen Ausgestaltung teilt das Bauteil 10 das Trägergas Tg auf die beiden Narkosemitteldosierer-Bauteile 38, 38.1 auf. Der Patient Pt wird daher mit einer Mischung von beiden Narkotisierungs-Gasgemischen Ng, Ng.1 versorgt.

Ein Narkosemittel-Sensor 27 misst ein Maß für die tatsächliche Konzentration von Narkosemittel in dem Narkotisierungs-Gasgemisch Ng, Ng.1, das durch die Zuführ-Fluidführungseinheit 30 hindurchfließt. Im Ausführungsbeispiel ist der Narkosemittel-Sensor 27 flussabwärts von dem oder jedem Narkosemitteldosierer-Bauteil 38, 38.1 und flussaufwärts vom Einspeisepunkt 28 angeordnet.

In einer Ausgestaltung verwendet das Steuergerät 11 ein Signal des Narkosemittel-Sensors 27, um die Konzentration von Narkosemittel Nm, Nm.1 in dem Narkotisierungs-Gasgemisch Ng, Ng.1 und damit in dem Beatmungs-Gasgemisch Bg, das zur patientenseitigen Koppeleinheit 1 fließt, zu regeln. Ein zeitlicher Verlauf oder ein Wert für die Soll-Konzentration con_{req}, con.1_{req} des Narkosemittels Nm, Nm.1 wird vorgegeben. Das Steuergerät 11 steuert den Narkosemitteldosierer 36, 36.1 oder auch ein Ventil mit dem Regelungsziel an, dass die tatsächliche Narkosemittel-Konzentration der Soll-Konzentration con_{req}, con.1_{req} gleicht oder folgt.

Im Ausführungsbeispiel ist in der Zuführ-Fluidführungseinheit 30 ein dritter Volumenfluss-Sensor 6.3 angeordnet. Der dritte Volumenfluss-Sensor 6.3 misst den Volumenfluss Vol'₃₀ durch die Zuführ-Fluidführungseinheit 30 hindurch.

Das Steuergerät 11 oder eine separate Auswerteeinheit leiten aus einem Signal des Narkosemittel-Sensors 27 und einem Signal des dritten Volumenfluss-Sensors 6.3 die Menge des Narkosemittels her, die in einer Zeitspanne durch die Zuführ-Fluidführungseinheit 30 geflossen ist. Der dritte Volumenfluss-Sensor 6.3 ist ebenfalls zwischen dem Narkosemitteldosierer-Bauteil 38, 38.1 und dem Einspeisepunkt 28 angeordnet. Die beiden Sensoren 27 und 6.3 sind in Reihe geschaltet. Der Volumenfluss-Sensor 6.3 kann so wie in Figur 2 und Figur 3 gezeigt flussabwärts vom Narkosemittel-Sensor 27 oder auch flussaufwärts vom Narkosemittel-Sensor 27 angeordnet sein.

Die Zuführ-Fluidführungseinheit 30 leitet das Narkotisierungs-Gasgemisch Ng, Ng.1 zu einem Einspeisepunkt 28, vgl. Figur 1. Das Narkotisierungs-Gasgemisch Ng, Ng.1 wird im Einspeisepunkt 28 in einen Beatmungskreislauf eingespeist.

Das Beatmungsgerät 100 stößt ein atembares Gasgemisch aus, welches Sauerstoff und mindestens ein Narkosemittel Nm, Nm.1 umfasst. Dieses Gasgemisch wird als das Beatmungs-Gasgemisch Bg bezeichnet und umfasst das Narkotisierungs-Gasgemisch Ng, Ng.1. Bevorzugt führt das Beatmungsgerät 100 eine Abfolge von Beatmungshüben (ventilation strokes) aus und stößt in jedem Beatmungshub jeweils eine Menge des Beatmungs-Gasgemischs Bg aus dem Gehäuse 12 aus. Das ausgestoßene Beatmungs-Gasgemisch Bg fließt durch die Inspirations-Fluidführungseinheit 3.1, 3.2 hindurch zum Y-Stück 7 und weiter durch die patientenseitige Fluidführungseinheit 2 hindurch und wird vom Patienten Pt mithilfe der patientenseitigen Koppeleinheit 1 eingeatmet.

Eine Fluidfördereinheit, beispielsweise ein Gebläse 4 oder eine Pumpe oder eine Kolben-Zylinder-Einheit, erzeugt einen Volumenfluss, beispielsweise einen zeitlich konstanten Volumenfluss, sowie einen Druck, beispielsweise einen zeitlich konstanten Druck. Der zeitlich konstante Druck liegt beispielsweise zwischen 10 mbar und 100 mbar. Die gezeigte Position der Fluidführungseinheit 4 ist nur beispielhaft zu verstehen.

Ein erster Druck-Sensor 5.1 misst den tatsächlichen Druck P_{3.1} im ersten Segment 3.1. Ein zweiter Drucksensor 5.2 misst den tatsächlichen Druck P_{3.2} im zweiten Segment 3.2. Ein dritter Drucksensor 5.3 misst den Druck in der patientenseitigen Fluidführungseinheit 2 und damit den Druck am Atemweg (pressure in airway, P_{AW}). Ein erster Volumenfluss-Sensor 6.1 misst den tatsächlichen Volumenfluss Vol'_{3.1} durch das erste Segment 3.1 hindurch. Ein zweiter Volumenfluss-Sensor 6.2 misst den tatsächlichen Volumenfluss Vol'_{3.2} durch das zweite Segment 3.2 hindurch. Genauer gesagt: Jeder Sensor 5.1, 5.2 misst jeweils eine Größe, die mit dem tatsächlichen Druck korreliert. Jeder Sensor 6.1, 6.2, 6.3 misst jeweils eine Größe, die mit den tatsächlichen Volumenfluss korreliert. Selbstverständlich brauchen nicht notwendigerweise alle diese Sensoren vorhanden sein.

Das Steuergerät 11 erhält jeweils ein Signal von den Sensoren 5.1, 5.2, 5.3 sowie 6.1, 6.2 und steuert eine Ventil-Anordnung 14 mit mindestens einem Ventil an. Die Ventil-Anordnung 14 befindet sich zwischen dem ersten Segment 3.1 und dem zweiten Segment 3.2. Das Steuergerät 11 führt in einer Ausgestaltung eine Regelung (closed-loop control) mit dem Regelungsziel durch, dass der tatsächliche zeitliche Verlauf des Volumenflusses Vol'_{3.2} zur patientenseitigen Koppeleinheit 1 oder der Druck P_{AW} an der patientenseitigen Koppeleinheit 1 einem vorgegebenen zeitlichen Sollverlauf folgt. Ein anderes oder zusätzliches mögliches Regelungsziel ist das folgende: Die Menge des Beatmungs-Gasgemischs Bg, das während einer Inspirationsphase zur patientenseitigen Koppeleinheit 1 fließt, soll gleich einer vorgegebenen Sollmenge sein, wobei als Sollmenge beispielsweise ein ermitteltes Tidalvolumen der Lunge des Patienten Pt vorgegeben wird.

Eine Exspirations-Fluidführungseinheit 8, beispielsweise ein weiterer Schlauch, führt vom Y-Stück 7 zurück zum Beatmungsgerät 100 - genauer gesagt: zurück zum Einspeisepunkt 28. Durch die Exspirations-Fluidführungseinheit 8 hindurch fließt das Gasgemisch, das der Patient Pt ausgeatmet hat. In der Exspirations-Fluidführungseinheit 8 ist bevorzugt ein end-exspiratorisches Ventil 9 angeordnet, welches sicherstellt, dass in der Lunge des Patienten Pt ein Mindestdruck erhalten bleibt. Bevorzugt entfernt ein nicht gezeigter CO2-Absorber in der Exspirations-Fluidführungseinheit 8 Kohlendioxid aus dem ausgeatmeten Gasgemisch.

In der Regel enthält das vom Patienten Pt ausgeatmete Gasgemisch Narkosemittel. Dieses Narkosemittel soll nicht in die Umgebung gelangen. Daher wird ein Beatmungskreislauf zwischen dem Beatmungsgerät 100 und der patientenseitigen Koppeleinheit 1 realisiert. Das vom Patienten Pt ausgeatmete Gasgemisch wird dank des Beatmungskreislaufs wieder in den Strom des Gasgemischs, den die Fluidfördereinheit 4 in Bewegung hält, eingespeist.

Im Einspeisepunkt 28 wird in diesen Beatmungskreislauf das Narkotisierungs-Gasgemisch Ng, Ng.1 eingespeist. Die Mischung aus dem eingespeisten Narkotisierungs-Gasgemisch Ng, Ng.1 und dem ausgeatmeten Gasgemisch, das durch die Exspirations-Fluidführungseinheit 8 hindurch zurückgeführt wird, bilden das Beatmungs-Gasgemisch Bg des Ausführungsbeispiels.

Aus diesem Beatmungskreislauf muss wenigstens zeitweise ein überschüssiges Gasgemisch (Abgas Ag) abgezweigt werden, vgl. Figur 1. Beispielhaft wird ein Überdruckventil 18 gezeigt. Eine Fluidführungseinheit 35 im Beatmungsgerät 100 führt vom Beatmungskreislauf oder vom Überdruckventil 18 zu einem Anschluss im Gehäuse 12 des Beatmungsgeräts 100. Eine Fluidführungseinheit 17, beispielsweise ein Schlauch, leitet das abgezweigte Gasgemisch Ag vom Beatmungsgerät 100 zur Wand W. Ein Stecker 15 am freien Ende der Fluidführungseinheit 17 lässt sich in eine Steckdose 16 in der Wand W einstecken. Das abgezweigte Gasgemisch Ag fließt durch die Fluidführungseinheiten 35 und 17, durch den Stecker 15 und die Steckdose 16 hindurch in ein nicht gezeigtes stationäres Aufnahmenetz hinter der Wand W.

Im Ausführungsbeispiel umfassen das Beatmungs-System und die Ansteuer-Anordnung alle drei Temperatursensoren der Temperatursensor-Gruppe, nämlich die folgenden:
- Ein Umgebungstemperatursensor 20 misst die Temperatur in einer Umgebung des Beatmungsgeräts 100.
- Ein narkosemitteldosierer-seitiger Temperatursensor 21 gehört zum Narkosemitteldosierer-Bauteil 38 und misst die Temperatur des Narkosemitteldosierer-Bauteils 38. Zum Narkosemitteldosierer-Bauteil 38.1 gehört ein weiterer, nicht gezeigter Narkosemitteldosierer-seitiger Temperatursensor.
- Ein aufnahme-seitiger (beatmungsgeräte-seitiger) Temperatursensor 22 misst die Temperatur an der Aufnahme 50 für das Narkosemitteldosierer-Bauteil 38. Ein weiterer aufnahme-seitiger Temperatursensor 22.1 misst die Temperatur an der Aufnahme 50.1 für das Narkosemitteldosierer-Bauteil 38.1.

Der narkosemitteldosierer-seitige Temperatursensor 21 steht in einem thermischen Kontakt mit derjenigen Oberfläche des Narkosemitteldosierer-Bauteils 38, die bei eingesetztem Narkosemitteldosierer-Bauteil 38 zur Aufnahme 50 hin zeigt. Daher misst der narkosemitteldosierer-seitige Temperatursensor 21 die Temperatur dieser Oberfläche. Diese Oberfläche fungiert im Ausführungsbeispiel als die Narkosemitteldosierer-Messposition. Das Entsprechende gilt für den weiteren narkosemitteldosierer-seitigen Temperatursensor des Narkosemitteldosierer-Bauteils 38.1. Bevorzugt umfasst auch bei der Ausgestaltung, bei der nur der Narkosemittelbehälter 37, 37.1 in die Aufnahme 50, 50.1 eingesetzt werden kann, das Narkosemitteldosierer-Bauteil 38, 38.1 den narkosemitteldosierer-seitigen Temperatursensor 21, 21.1.

Der aufnahme-seitige Temperatursensor 22, 22.1 fungiert als der beatmungsgeräte-seitige Temperatursensor des Ausführungsbeispiels und steht in einem thermischen Kontakt mit einer Oberfläche der Aufnahme 50, 50.1, wobei dieser Oberfläche zu einem eingesetzten Narkosemitteldosierer-Bauteil 38, 38.1 hin zeigt und als die Beatmungsgeräte-Messposition fungiert. Bevorzugt steht der aufnahme-seitige Temperatursensor 22, 22.1 zusätzlich in einem thermischen Kontakt mit einer Wand der Zuführ-Fluidführungseinheit 30. Bevorzugt ist der aufnahme-seitige Temperatursensor 22, 22.1 zwischen einer aufnahme-seitigen pneumatischen Koppelstelle in der Aufnahme 50, 50.1 und dem Narkosemittel-Sensor 27 angeordnet. Durch diese Koppelstelle hindurch fließt das Narkotisierungs-Gasgemisch Ng, Ng.1 in das Beatmungsgerät 100 hinein. In einer Ausgestaltung umfasst der Narkosemittel-Sensor 27 eine Bodenplatte aus einem Metall, und der aufnahme-seitige Temperatursensor 22, 22.1 misst die Temperatur der Bodenplatte. Die Erfinder haben in internen Versuchen herausgefunden, dass die Temperatur der Bodenplatte nur wenig von der Temperatur der zum Narkosemitteldosierer-Bauteil 38, 38.1 hin zeigenden Oberfläche der Aufnahme 50, 50.1 abweicht.

Die Erfinder haben intern folgendes Problem identifiziert: Gasförmiges Narkosemittel Nm, Nm.1 im erzeugten Narkotisierungs-Gasgemisch Ng bzw. Ng.1 kann kondensieren und sich auf einer Wand der Beatmungs-Anordnung absetzen, wenn das Narkosemittel Nm, Nm.1 eine zu hohe Konzentration aufweist. Dieses Kondensieren kann im Narkosemitteldosierer-Bauteil 38, 38.1 und / oder im Beatmungsgerät 100 auftreten. Kondensiertes Narkosemittel Nm, Nm.1 in der Zuführ-Fluidführungseinheit 30 kann in den Narkosemittel-Sensor 27 hineinfließen und ein Messergebnis des Narkosemittel-Sensors 27 verfälschen. Insbesondere kann kondensiertes Narkosemittel Nm, Nm.1 dazu führen, dass der Narkosemittel-Sensor 27 eine zu geringe Narkosemittel-Konzentration misst und daher der Patient Pt mit zu viel Narkosemittel Nm, Nm.1 versorgt wird. Das Kondensieren von Narkosemittel Nm, Nm.1 kann auch dazu führen, dass der Patient Pt zu wenig Narkosemittel Nm, Nm.1 erhält Die Erfindung reduziert signifikant das Risiko, dass gasförmiges Narkosemittel Nm, Nm.1 kondensiert.

Weiter oben wurde bereits dargelegt, dass ein Benutzer mithilfe der Bedieneinheit 26 eine Soll-Konzentration con_{req}, con.1_{req} des Narkosemittels Nm bzw. Nm.1 im erzeugten Narkotisierungs-Gasgemisch Ng bzw. Ng.1 vorgeben kann. Diese Soll-Konzentration con_{req}, con.1_{req} liegt in einem Wertebereich, der nach oben durch eine obere Konzentrations-Schranke conₘₐₓ bzw. con.1ₘₐₓ begrenzt wird. Im Ausführungsbeispiel kann der Benutzer mit der Bedieneinheit 26 für die beiden Narkosemitteldosierer-Bauteile 38 und 38.1 jeweils eine Soll-Konzentration con_{req}, con.1_{req} vorgeben, also insgesamt zwei Soll-Konzentrationen con_{req}, con.1_{req}, wobei diese beiden Soll-Konzentrationen con_{req}, con.1_{req} sich voneinander unterscheiden können.

Eine denkbare Abhilfe für das oben genannte Problem, dass Narkosemittel Nm, Nm.1 kondensieren kann, wäre die folgende: Die obere Konzentrations-Schranke conₘₐₓ, con.1ₘₐₓ wird so klein vorgegeben, dass in keiner Situation Narkosemittel Nm, Nm.1 kondensieren kann. Dies würde aber die Einsatzmöglichkeiten des Beatmungsgeräts 100 einschränken. Eine weitere denkbare Abhilfe wäre, die gesamte Zuführ-Fluidführungseinheit 30 oder wenigstens ein Segment zu beheizen. Die Erfindung lässt sich in Kombination mit einer solchen Heizung einsetzen. Die Erfindung zeigt aber einen anderen oder zusätzlichen Weg auf, um wenigstens weitgehend zu verhindern, dass Narkosemittel kondensiert.

Die Erfinder haben intern folgende mögliche Ursache für ein Kondensieren festgestellt: Häufig wird ein Narkosemitteldosierer-Bauteil 38, 38.1 in einem Lagerraum oder sonstigem Lagerbereich vorrätig gehalten und bei Bedarf aus diesem Lagerbereich geholt und in eine Aufnahme 50, 50.1 des Beatmungsgeräts 100 eingesetzt. Insbesondere um zu verhindern, dass Narkosemittel Nm, Nm.1 bereits im Narkosemitteldosierer-Bauteil 38, 38.1 verdampft oder verdunstet, weist der Lagerbereich eine relativ geringe Temperatur auf, insbesondere eine geringere Temperatur als der Raum, in dem der Patient Pt mit dem Narkotisierungs-Gasgemisch Ng bzw. Ng.1 versorgt wird. Außerdem wird häufig auch das Beatmungsgerät 100 in einem kühleren Lagerraum vorrätig gehalten. Wenn später das Narkosemitteldosierer-Bauteil 38, 38.1 in die Aufnahme 50, 50.1 eingesetzt wird und anschließend das Narkotisierungs-Gasgemisch Ng bzw. Ng.1 erzeugt wird, so weisen anfangs das Narkosemitteldosierer-Bauteil 38, 38.1 und optional auch die Aufnahme 50, 50.1 noch die Temperatur des Lagerbereichs auf und erwärmen sich erst allmählich, nämlich in einer Aufwärmphase, auf die Umgebungstemperatur in der Umgebung des verwendeten Beatmungsgeräts 100.

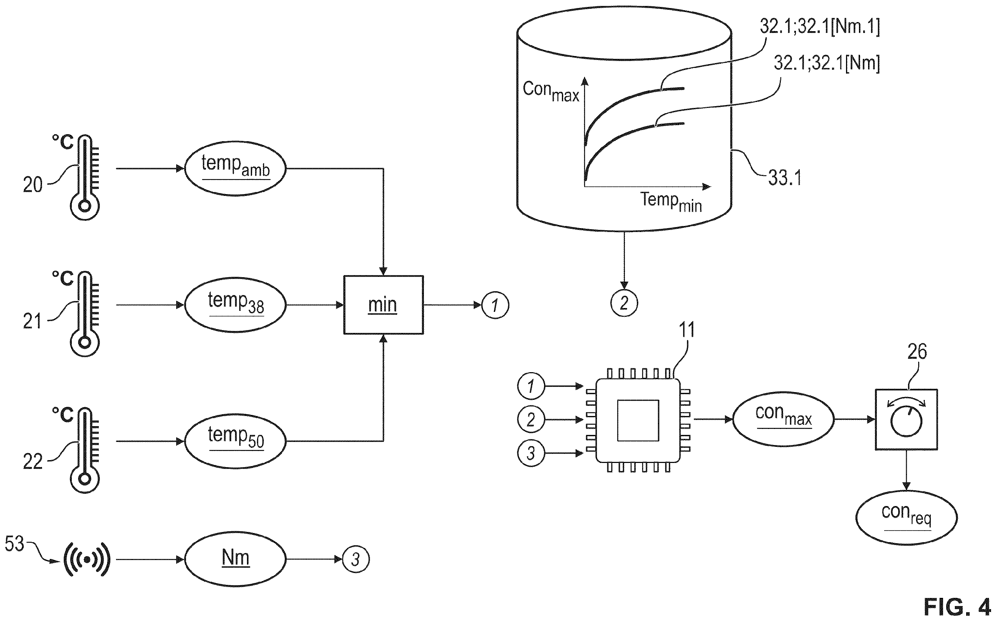

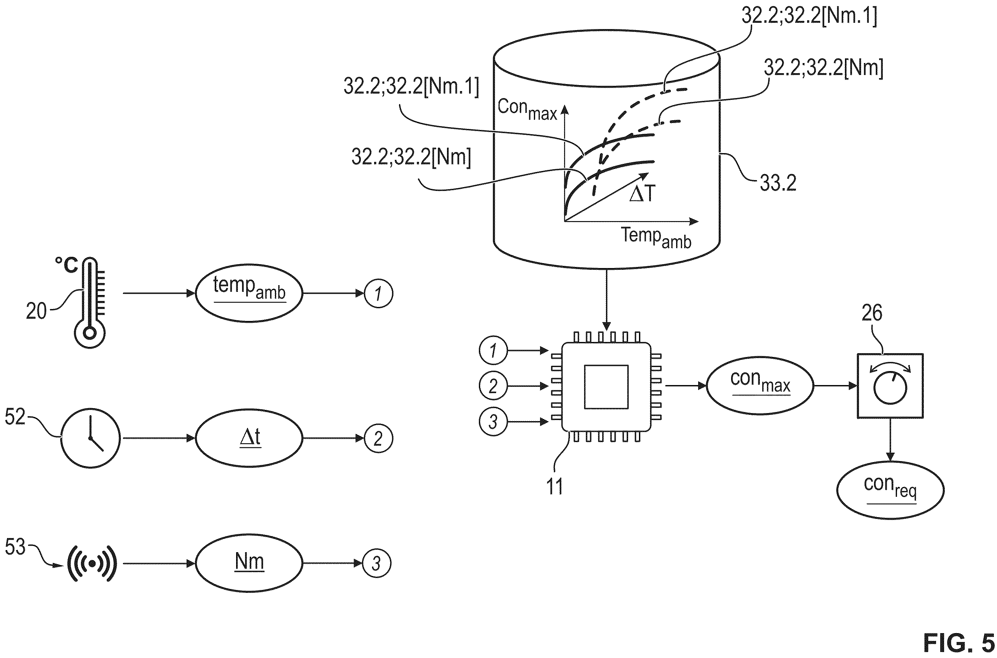

Erfindungsgemäß berechnet das Steuergerät 11 die obere Konzentrations-Schranke conₘₐₓ, con.1ₘₐₓ für den Wertebereich, aus dem der Benutzer mithilfe der Bedieneinheit 26 eine gewünschte Soll-Konzentration con_{req}, con.1_{req} auswählen und dem Narkosemitteldosierer-Bauteil 38, 38.1 vorgeben kann. Figur 4 und Figur 5 veranschaulichen schematisch zwei Ausgestaltungen, wie das Steuergerät 11 dies durchführt. Bei der Ausgestaltung, die in Figur 4 gezeigt wird, werden die drei Temperatursensoren 20, 21, 22 verwendet. Bei der Ausgestaltung gemäß Figur 5 wird nur der Umgebungstemperatur-Sensor 20 verwendet. Möglich ist natürlich, auch bei der Ausgestaltung gemäß Figur 5 drei Temperatursensoren 20, 21, 22 zu verwenden.

Anmerkung: In dieser Darstellung wird der Name einer physikalischen Größe mit einem Großbuchstaben am Anfang bezeichnet, der Name eines gemessenen Werts dieser physikalischen Größe mit einem Kleinbuchstaben.

Der Umgebungstemperatur-Sensor 20 liefert einen gemessenen Wert temp_{amb} für die Umgebungstemperatur Temp_{amb}. Der narkosemitteldosierer-seitige Temperatursensor 21 liefert einen gemessenen Wert temp₃₈ für die Temperatur Temp₃₈ an der zur Aufnahme 50 zeigenden Oberfläche des Narkosemitteldosierer-Bauteils 38. Der aufnahme-seitige Temperatursensor 22 liefert einen gemessenen Wert temp₅₀ für die Temperatur Temp₅₀ an der zum Narkosemitteldosierer-Bauteil 38 zeigenden Oberfläche der Aufnahme 50. Bevorzugt messen die drei Temperatursensoren 20, 21, 22 die jeweilige Temperatur wiederholt, beispielsweise mit einer festen Abtastrate. Die Systemuhr 52 liefert einen gemessenen Wert ΔT für die Zeitspanne Δt, die verstrichen ist, seitdem das Narkosemitteldosierer-Bauteil 38 in die Aufnahme 50 eingesetzt ist. Ein Lesegerät 53 erfasst eine Kennung auf einer Oberfläche oder in einem Datenspeicher des Narkosemitteldosierer-Bauteils 38. Diese Kennung legt fest, welches Narkosemittel Nm das vom Narkosemitteldosierer-Bauteil 38 bereitgestellte Narkotisierungs-Gasgemisch Ng enthält.

In der Ausgestaltung gemäß Figur 4 berechnet eine Funktionseinheit min das Minimum der drei gemessenen Temperaturen temp_{amb}, temp₃₈ und temp₅₀. Eine andere Rechenvorschrift, um die drei gemessenen Temperaturen temp_{amb}, temp₃₈ und temp₅₀ zu einem Wert zusammenzufassen, ist ebenfalls möglich, beispielsweise ein gewichtetes Mittel oder der mittlere der drei Werte.

Bei der Ausgestaltung gemäß Figur 4 (drei Temperatursensoren 20, 21, 22) ist in rechnerauswertbarer Form ein funktionaler Zusammenhang 32.1 vorgegeben und ist in einem Datenspeicher 33.1 abgespeichert. Dieser funktionale Zusammenhang 32.1 beschreibt die obere Konzentrations-Schranke Conₘₐₓ als Funktion des Temperatur-Minimums Tempₘᵢₙ = min(Temp_{amb}, Temp₃₈, Temp₅₀). Der funktionale Zusammenhang 32.1 ist so ausgestaltet, dass die obere Konzentrations-Schranke Conₘₐₓ umso größer ist, je größer das Temperatur-Minimum Tempₘᵢₙ ist.

Dieser funktionale Zusammenhang 32.1 wird vor einem Einsatz der Beatmungs-Anordnung aufgestellt. Der funktionale Zusammenhang 32.1 umfasst im Ausführungsbeispiel für jedes in Betracht kommenden Narkosemittel Nm, Nm.1 jeweils einen funktionalen Einzel-Zusammenhang 32.1[Nm], 32[Nm.1]. Jeder funktionale Einzel-Zusammenhang 32.1 [Nm], 32[Nm.1] für ein Narkosemittel Nm, Nm.1 ist so ausgestaltet, dass die obere Konzentrations-Schranke Conₘₐₓ für dieses Narkosemittel Nm, Nm.1 umso größer ist, je größer das Temperatur-Minimum Tempₘᵢₙ ist.

Der physikalische Hintergrund, um die funktionalen Einzel-Zusammenhänge aufzustellen, ist der folgende: Wie oben bereits erwähnt, weist jedes Narkosemittel eine Sättigungs-Konzentration auf. Je größer die Temperatur eines Gasgemisch ist, welches dieses Narkosemittel enthält, umso größer ist die Sättigungs-Konzentration für dieses Narkosemittel. Beispielhafte Werte sind:

| *Narkosemittel* | *Sättigungs-Konzentration bei 10* °*C in [Vol.-%]* | *Sättigungs-Konzentration bei 20* °*C in [Vol.-%]* |
|---|---|---|
| Isofluran | 19 | 31 |
| Sevofluran | 12 | 20 |
| Desfluran | 58 | 88 |

Diese Werte sind bekannt und werden vorgegeben. Außerdem wird ein Sicherheitsabschlag vorgegeben. Ausgenutzt wird die Tatsache, dass die Temperatur des Narkotisierungs-Gasgemisch Ng, Ng.1 mit dem Narkosemittel Nm, Nm.1 mindestens so groß ist wie das Temperatur-Minimum Tempₘᵢₙ abzüglich des vorgegebenen Sicherheitsabschlags. Eine Kalibrierungsvorrichtung erzeugt vorab unter Verwendung der vorgegebenen Sättigungs-Konzentrationen und des Sicherheitsabschlags die funktionalen Einzel-Zusammenhänge 32.1[Nm], 32[Nm.1].

Das Steuergerät 11 wendet den funktionalen Zusammenhang 32.1 auf den berechneten Wert tempₘᵢₙ für das Temperatur-Minimum Tempₘᵢₙ an, welches die Funktionseinheit min liefert. In dem Beispiel, das in Figur 4 und Figur 5 gezeigt wird, berechnet das Steuergerät 11 einen Wert conₘₐₓ für die obere Konzentrations-Schranke Conₘₐₓ, wobei der Wert conₘₐₓ sich auf das Narkosemitteldosierer-Bauteil 38 bezieht, das in die Aufnahme 50 eingesetzt ist und das Narkotisierungs-Gasgemisch Ng mit dem Narkosemittel Nm liefert. Auf die gleiche Weise wird der Wert con.1ₘₐₓ berechnet.

Das Steuergerät 11 empfängt vom Lesegerät 53 die Information, dass das eingesetzte Narkosemitteldosierer-Bauteil 38 ein Narkotisierungs-Gasgemisch Ng mit dem Narkosemittel Nm zu erzeugen vermag. Das Steuergerät 11 wählt den funktionalen Einzel-Zusammenhang 32.1[Nm] für dieses Narkosemittel Nm aus. Das Steuergerät 11 wendet den ausgewählten funktionalen Einzel-Zusammenhang 32.1[Nm] auf den gemessenen Wert temp_{amb} der Umgebungstemperatur Temp_{amb} an. Durch die Anwendung erzeugt das Steuergerät 11 einen Wert conₘₐₓ für die obere Konzentrations-Schranke Conₘₐₓ.

Dieser Wert conₘₐₓ für die obere Konzentrations-Schranke Conₘₐₓ wird an die Bedieneinheit 26 übermittelt. Bevorzugt ist die Bedieneinheit 26 so ausgestaltet, dass dem Benutzer ausschließlich der Wertebereich von Null bis zum übermittelten Wert conₘₐₓ der oberen Konzentrations-Schranke Conₘₐₓ zur Auswahl angeboten wird. Dadurch wird das Ereignis vermieden, dass der Benutzer einen Wert con_{req} für die gewünschte Narkosemittel-Konzentration auswählt, dieser Wert con_{req} aber nicht realisiert wird, weil er größer als der übermittelte Wert conₘₐₓ für die obere Konzentrations-Schranke Conₘₐₓ ist.

Bevorzugt misst jeder Temperatursensor 20, 21, 22 wiederholt die jeweilige Temperatur Temp_{amb}, Temp₃₈, Temp₅₀. In der Regel bleibt die Umgebungstemperatur Temp_{amb} annähernd konstant. Hingegen steigen häufig die gemessenen Temperaturen Temp₃₈ und Temp₅₀ an, weil die Temperatur Temp₅₀ des Beatmungsgeräts 100 und die Temperatur Temp₃₈ des Narkosemitteldosierer-Bauteils 38 sich an die Umgebungstemperatur Temp_{amb} anpassen. Diesen Anstieg bildet die Anwendung des funktionalen Zusammenhang 38.1 bzw. des ausgewählten funktionalen Einzel-Zusammenhangs nach. Eine Wirkung ist die folgende: Zu Beginn eines Einsatzes ist der Wert der oberen Konzentrations-Schranke conₘₐₓ kleiner als nach einer längeren Einsatzzeit. Der Vorgang, dass sich die Temperatur Temp₅₀ des Beatmungsgerätes 100 und die Temperatur Temp₃₈ des Narkosemitteldosierer-Bauteils 38 an die Umgebungstemperatur Temp_{amb} anpassen, führt dazu, dass die Narkosemittel-Konzentration vergrößert werden kann, ohne dass eine große Gefahr der Kondensation auftritt.

In der Ausgestaltung gemäß Figur 5 wird in rechnerauswertbarer Form ein funktionaler Zusammenhang 32.2 vorgegeben und ist in einem Datenspeicher 33.2 abgespeichert. Der funktionale Zusammenhang 32.2 umfasst für jedes Narkosemittel Nm, Nm.1 jeweils einen funktionalen Einzel-Zusammenhang 32.2[Nm], 32.2[Nm.1]. Jeder funktionale Zusammenhang Einzel-Zusammenhang 32.2[Nm], 32.2[Nm.1] beschreibt die obere Konzentrations-Schranke Conₘₐₓ als Funktion der gemessenen Umgebungstemperatur Temp_{amb} und der Zeitspanne ΔT, die seit dem Einsetzen des Narkosemitteldosierer-Bauteils 38 in die Aufnahme 50 verstrichen ist. Jeder funktionale Einzel-Zusammenhang 32.2[Nm], 32.2[Nm.1] ist also ein dreidimensionaler Zusammenhang. Wiederum ist für jeden Wert Δt der Zeitspanne ΔT die obere Konzentrations-Schranke Conₘₐₓ umso größer, je größer die gemessene Umgebungstemperatur Temp_{amb} ist. Mit anderen Worten: Bei gleichbleibender Zeitspanne ist die oberen Konzentrations-Schranke Conₘₐₓ umso größer, je größer die gemessene Umgebungstemperatur Temp_{amb} ist. Andererseits ist jeder funktionale Einzel-Zusammenhang 32.2[Nm], 32.2[Nm.1] bevorzugt wie folgt vorgegeben: Bei jedem Wert temp_{amb} der Umgebungstemperatur Temp_{amb} steigt in einer Aufwärmphase die obere Konzentrations-Schranke Conₘₐₓ an und bleibt dann konstant. Diese Ausgestaltung berücksichtigt folgende Gegebenheit: In der Aufwärmphase passen sich die Temperatur Temp₅₀ des Beatmungsgeräts 100 und damit die der Aufnahme 50 und die Temperatur Temp₃₈ des Narkosemitteldosierer-Bauteils 38 an die gleichbleibende Umgebungstemperatur Temp_{amb} an und bleiben danach gleich.

Wiederum generiert bevorzugt eine Kalibrierungsvorrichtung vorab jeden funktionalen Einzel-Zusammenhang 32.2[Nm], 32.2[Nm.1]. Einerseits wird für jedes Narkosemittel Nm, Nm.1 und für verschiedene Temperaturen jeweils eine Sättigungs-Konzentration vorgegeben. Andererseits wird bevorzugt eine untere Schranke für die Temperatur vorgegeben, welche ein Narkosemitteldosierer-Bauteil 38 und ein Beatmungsgerät 100 vor einem Einsatz haben können. Außerdem wird empirisch eine Stichprobe erzeugt, wobei die Stichprobe mehrere Stichprobenelemente umfasst. Jedes Stichprobenelement wird wie folgt erzeugt: Das Narkosemitteldosierer-Bauteil 38 und das Beatmungsgerät 100 - genauer gesagt: die Aufnahme 50 - werden auf jeweils eine bestimmte anfängliche Temperatur gebracht. Außerdem wird eine bestimmte Umgebungstemperatur Temp_{amb} hergestellt. Das Narkosemitteldosierer-Bauteil 38 wird in die Aufnahme 50 eingesetzt. Gemessen wird der zeitliche Verlauf der Temperatur Temp₃₈ des eingesetzten Narkosemitteldosierer-Bauteils 38 und der zeitliche Verlauf der Temperatur Temp₅₀ in der Nähe der Aufnahme 50. In der Regel hängt der zeitliche Verlauf der Temperatur Temp₃₈ des eingesetzten Narkosemitteldosierer-Bauteils 38 nicht wesentlich davon ab, welches Narkosemittel Nm, Nm.1 in das Narkosemitteldosierer-Bauteil 38 gefüllt ist, sodass dieser zeitliche Verlauf jedes in Betracht kommenden Narkosemittel Nm, Nm.1 gilt. Aus den beiden zeitlichen Temperatur-Verläufen und aus einem vorgegebenen Sicherheitsabschlag wird für jedes Narkosemittel Nm, Nm.1 jeweils ein zeitlicher Verlauf der Sättigungs-Konzentration hergeleitet. Hieraus wird für jedes Narkosemittel Nm, Nm.1 jeweils ein funktionaler Einzel-Zusammenhang 32.2[Nm], 32.2[Nm.1] hergeleitet und abgespeichert.

### Bezugszeichenliste

| | |
|---|---|
| 1 | patientenseitige Koppeleinheit in Form einer Atemmaske, mit der Fluidführungseinheit 2 verbunden, am Körper des Patienten Pt angeordnet |
| 2 | patientenseitige Fluidführungseinheit, verbindet das Y-Stück 7 mit der patientenseitigen Koppeleinheit 1 |
| 3.1 | erstes Segment der Inspirations-Fluidführungseinheit, führt von der Fluidfördereinheit 4 zur Ventil-Anordnung 14 |
| 3.2 | zweites Segment der Inspirations-Fluidführungseinheit, führt von der Ventil-Anordnung 14 zum Y-Stück 7 |
| 4 | Fluidfördereinheit in Form eines Gebläses, stößt ein Gasgemisch in das erste Segment 3.1 hinein aus, ist mit dem Versorgungsanschluss 13 verbunden |
| 5.1 | erster Drucksensor, misst ein Maß für den tatsächlichen Druck P_{3.1} im ersten Segment 3.1, wobei der Druck in der Regel von der Fluidfördereinheit 4 erzeugt wird |
| 5.2 | zweiter Drucksensor, misst ein Maß für den tatsächlichen Druck P_{3.2} im zweiten Segment 3.2 |
| 5.3 | dritter Drucksensor, misst ein Maß für den tatsächlichen Druck in der patientenseitigen Koppeleinheit 1, in der Regel den Atemwegsdruck P_{AW} |
| 6.1 | erster Volumenfluss-Sensor, misst ein Maß für den tatsächlichen Volumenfluss Vol'_{3.1} durch das erste Segment 3.1 |
| 6.2 | zweiter Volumenfluss-Sensor, misst ein Maß für den tatsächlichen Volumenfluss Vol'3.2 durch das zweite Segment 3.2 |
| 6.3 | dritter Volumenfluss-Sensor, misst ein Maß für den tatsächlichen Volumenfluss Vol'₃₀ durch die Zuführ-Fluidführungseinheit 30 |
| 7 | Y-Stück, verbindet die Fluidführungseinheiten 3.2 und 8 auf der einen Seite mit der patientenseitigen Fluidführungseinheit 2 auf der anderen Seite |
| 8 | Exspirations-Fluidführungseinheit, führt vom Y-Stück 7 zurück zum Beatmungsgerät 100 |
| 9 | end-exspiratorisches Ventil in der Exspirations-Fluidführungseinheit 8 |
| 10 | pneumatisches Schaltventil, leitet das Trägergas Tg aus dem Trägergas-Mischer 24 wahlweise zum Narkosemitteldosierer-Bauteil 38 oder zum Narkosemitteldosierer-Bauteil 38.1 oder teilt das Trägergas Tg auf die beiden Narkosemitteldosierer-Bauteile 38, 38.1 auf |
| 11 | signalverarbeitendes Steuergerät, empfängt und verarbeitet Signale von den Sensoren 5.1, 5.2, 5.3 sowie 6.1, 6.2, 6.3, steuert die Ventil-Anordnung 14 und den Trägergas-Mischer 24 an, berechnet die Soll-Konzentration des Narkosemittels Nm |
| 12 | Gehäuse des Beatmungsgeräts 100, weist die Aufnahmen 50, 50.1 auf |
| 13 | Versorgungsanschluss-Anordnung des Beatmungsgeräts 100, mit der Fluidfördereinheit 4 verbunden, umfasst die Versorgungsanschlüsse 13.1, 13.2, 13.3 für die drei möglichen Bestandteile des Trägergases Tg |
| 13.1 | Versorgungsanschluss für Atemluft, gehört zur Versorgungsanschluss-Anordnung 13 |
| 13.2 | Versorgungsanschluss für Lachgas (N2O), gehört zur Versorgungsanschluss-Anordnung 13 |
| 13.3 | Versorgungsanschluss für reinen Sauerstoff, gehört zur Versorgungsanschluss-Anordnung 13 |
| 14 | Ventil-Anordnung, zwischen den Segmenten 3.1 und 3.2 angeordnet, umfasst mindestens ein ansteuerbares Ventil |
| 15 | Stecker am freien Ende der Fluidführungseinheit 17, lässt sich in die Steckdose 16 einstecken |
| 16 | Steckdose in der Wand W, nimmt den Stecker 15 auf, mit einer Senke hinter der Wand W verbunden |
| 17 | Fluidführungseinheit, führt von der Fluidführungseinheit 35 zum Stecker 15 |
| 18 | Überdruckventil im Beatmungskreislauf, Anfangspunkt der Fluidführungseinheit 35 |
| 20 | Umgebungstemperatur-Sensor, liefert die Umgebungstemperatur Temp_{amb} |
| 21 | narkosemitteldosierer-seitiger Temperatursensor, liefert die Temperatur Temp₃₈ |
| 22 | aufnahme-seitiger Temperatursensor, liefert die Temperatur Temp₅₀ an der Aufnahme 50 für das Narkosemitteldosierer-Bauteil 38 |
| 22.1 | weiterer aufnahme-seitiger Temperatursensor, liefert die Temperatur an der Aufnahme 50.1 für das weitere Narkosemitteldosierer-Bauteil 38.1 |
| 24 | Trägergas-Mischer, erzeugt das Trägergas Tg aus den Gasen, die aus den Versorgungsanschlüssen 13.1, 13.2, 13.3 fließen, mit der Versorgungsanschluss-Anordnung 13 verbunden |
| 25 | Bedieneinheit für den Trägergas-Mischer 24, ermöglicht es einem Benutzer, das Mischungsverhältnis im Trägergas Tg festzulegen |
| 26 | Bedieneinheit für den Narkosemitteldosierer 36, 36.1, ermöglicht es einem Benutzer, eine Soll-Konzentration des Narkosemittels Nm, Nm.1 im Narkotisierungs-Gasgemisch Ng bzw. Ng.1 vorzugeben. |
| 27 | Narkosemittel-Sensor, misst die tatsächliche Konzentration von Narkosemittel in dem Narkotisierungs-Gasgemisch Ng, Ng.1, das durch die Zuführ-Fluidführungseinheit 30 hindurchfließt |
| 28 | Einspeisepunkt, in dem das Narkotisierungs-Gasgemisch Ng, Ng.1, das durch die Zuführ-Fluidführungseinheit 30 hindurchgeflossen ist, in den Beatmungskreislauf eingespeist wird |
| 29 | Heizung in der Einspeisekammer des Narkosemitteldosierers 36 |
| 29.1 | Heizung in der Einspeisekammer des weiteren Narkosemitteldosierers 36.1 |
| 30 | Zuführ-Fluidführungseinheit, führt vom Narkosemitteldosierer-Bauteil 38, 38.1 zum Einspeisepunkt 28 |
| 32.1 | funktionaler Zusammenhang: obere Konzentrations-Schranke Conₘₐₓ als Funktion der Temperatur |
| 32.2 | funktionaler Zusammenhang: obere Konzentrations-Schranke als Funktion der Temperatur und der Zeitspanne ΔT seit dem Einsetzen des Narkosemitteldosierer-Bauteils 38 in die Aufnahme 50 |
| 33.1 | Datenspeicher mit dem funktionalen Zusammenhang 32.1 |
| 33.2 | Datenspeicher mit dem funktionalen Zusammenhang 32.2 |
| 35 | Fluidführungseinheit im Beatmungsgerät 100, verbindet den Beatmungskreislauf mit der Fluidführungseinheit 17 |
| 36 | Narkosemitteldosierer, erhält ein Trägergas Tg vom Versorgungsanschluss 13 und flüssiges Narkosemittel Nm vom Narkosemittelbehälter 37, erzeugt in einer Einspeisekammer ein Gemisch Ng aus dem Trägergas Tg und gasförmigem Narkosemittel, umfasst die Heizung 29, gehört in einer Realisierungsform zum Narkosemitteldosierer-Bauteil 38 |
| 36.1 | weiterer Narkosemitteldosierer, erhält ein Trägergas Tg vom Versorgungsanschluss 13 und flüssiges Narkosemittel Nm.1 vom weiteren Narkosemittelbehälter 37.1, erzeugt in einer |
| | Einspeisekammer ein Gemisch Ng.1 aus dem Trägergas Tg und gasförmigem Narkosemittel, umfasst die Heizung 29.1, gehört in einer Realisierungsform zum weiteren Narkosemitteldosierer-Bauteil 38.1 |
| 37 | Behälter mit flüssigem Narkosemittel Nm, lässt sich in einer Realisierungsform in das Gehäuse 12 einsetzen und wieder aus dem Gehäuse 12 entnehmen, gehört in einer anderen Realisierungsform zum Narkosemitteldosierer-Bauteil 38 |
| 37.1 | weiterer Behälter mit flüssigem Narkosemittel Nm.1 |
| 38 | Narkosemitteldosierer-Bauteil, umfasst den Narkosemitteldosierer 36 und den Narkosemittelbehälter 37, lässt sich in einer Realisierungsform als Ganzes in das Gehäuse 12 einsetzen und wieder aus dem Gehäuse 12 entnehmen |
| 38.1 | weiteres Narkosemitteldosierer-Bauteil, umfasst den Narkosemitteldosierer 36.1 und den Narkosemittelbehälter 37.1 |
| 49 | Trägergas-Zuführeinheit, führt vom Trägergas-Mischer 24 zum Narkosemitteldosierer 36 |
| 50 | Aufnahme im Gehäuse 12 für den Narkosemittelbehälter 37 oder für das Narkosemitteldosierer-Bauteil 38 |
| 50.1 | weitere Aufnahme im Gehäuse 12 für den weiteren Narkosemittelbehälter 37.1 oder für das weitere Narkosemitteldosierer-Bauteil 38.1 |
| 51 | Kontaktschalter, detektiert, ob in die Aufnahme 50 ein Narkosemitteldosierer-Bauteil 38 eingesetzt ist oder nicht |
| 52 | Systemuhr, misst die Zeitspanne ΔT, die seit dem Einsetzen des Narkosemitteldosierer-Bauteils 38 in die Aufnahme 50 verstrichen ist |
| 53 | Lesegerät an der Aufnahme 50, liest eine Kennzeichnung auf einem eingesetzten Narkosemitteldosierer-Bauteil 38, welche die Art des Narkosemittels kennzeichnet |
| 60 | Zusammenführeinheit, leitet das Narkotisierungs-Gasgemisch Ng vom Narkosemitteldosierer-Bauteil 38 und das weitere Narkotisierungs-Gasgemisch Ng.1 vom weiteren Narkosemitteldosierer-Bauteil 38.1 in die Zuführ-Fluidführungseinheit 30 |
| 100 | Beatmungsgerät, umfasst das Gehäuse 12 mit den Aufnahmen 50, 50.1, den Trägergas-Mischer 24, die Fluidfördereinheit 4, das Schaltventil 20, die Sensoren 5.1, 5.2, 6.1, 6.2, 6.3, die Fluidführungseinheiten 30, 3.1, 3.2 und das Steuergerät 11 |
| Ag | Abgas (überschüssiges Gasgemisch), wird aus dem Beatmungskreislauf abgezweigt und durch den Stecker 15 hindurch abgesaugt |
| Bg | Beatmungs-Gasgemisch, fungiert als das atembare Gasgemisch, umfasst das Narkotisierungs-Gasgemisch Ng, Ng.1 und das Trägergas Tg, wird von der Inspirations-Fluidführungseinheit vom Einspeisepunkt 28 zur patientenseitigen Koppeleinheit 1 geleitet |
| Conₘₐₓ | obere Konzentrations-Schranke für die Konzentration des Narkosemittels Nm im Narkotisierungs-Gasgemisch Ng, zugleich obere Grenze des Wertebereichs, aus dem der Benutzer mithilfe der Bedieneinheit 26 eine gewünschte Soll-Konzentration con_{req} auswählen kann |
| conₘₐₓ | Wert der oberen Konzentrations-Schranke Conₘₐₓ für das Narkotisierungs-Gasgemisch Ng |
| **con.1**ₘₐₓ | Wert der oberen Konzentrations-Schranke Conₘₐₓ für das Narkotisierungs-Gasgemisch Ng.1 |
| **con_{req}** | Soll-Konzentration des Narkosemittels im Narkotisierungs-Gasgemisch Ng, hängt von einer Benutzervorgabe ab, ist höchstens gleich dem Wert conₘₐₓ |
| **con.1**_{req} | Soll-Konzentration des Narkosemittels im Narkotisierungs-Gasgemisch Ng.1, hängt von einer Benutzervorgabe ab, ist höchstens gleich dem Wert con.1ₘₐₓ |
| Ng | Narkotisierungs-Gasgemisch, umfasst das Trägergas Tg und das Narkosemittel Nm, wird vom Narkosemitteldosierer-Bauteil 38 bereitgestellt und von der Zuführ-Fluidführungseinheit 30 zum Einspeisepunkt 28 geleitet |
| Ng.1 | weiteres Narkotisierungs-Gasgemisch, umfasst das Trägergas Tg und das Narkosemittel Nm.1, wird vom weiteren Narkosemitteldosierer-Bauteil 38.1 bereitgestellt und von der Zuführ-Fluidführungseinheit 30 zum Einspeisepunkt 28 geleitet |
| Nm | flüssiges Narkosemittel, stammt aus dem Narkosemittelbehälter 37 |
| Nm.1 | flüssiges Narkosemittel, stammt aus dem weiteren Narkosemittelbehälter 37.1 |
| ΔT | Zeitspanne, die seit dem Einsetzen des Narkosemitteldosierer-Bauteils 38 in die Aufnahme 50 verstrichen ist, wird von der Systemuhr 52 gemessen |
| Δt | gemessener Wert der Zeitspanne ΔT |
| Temp_{amb} | Umgebungstemperatur, wird vom Umgebungstemperatur-Sensor 20 gemessen |
| temp_{amb} | gemessener Wert der Umgebungstemperatur Temp_{amb} |
| Temp₃₈ | Temperatur des Narkosemitteldosierer-Bauteils 38, wird vom narkosemitteldosierer-seitigen Temperatursensor 21 gemessen |
| temp₃₈ | gemessener Wert der Temperatur Temp₃₈ |
| Temp₅₀ | Temperatur der Aufnahme 50, wird vom aufnahme-seitigen Temperatursensor 22 gemessen |
| temp₅₀ | gemessener Wert der Temperatur Temp₅₀ |
| Tg | Trägergas, wird vom Trägergas-Mischer 24 bereitgestellt und durch die Trägergas-Fluidführungseinheit 49 zum Narkosemitteldosierer 36, 36.1 geleitet |
| Vol'_{3.1} | Volumenfluss durch das erste Segment 3.1, vom ersten Volumenfluss-Sensor 6.1 gemessen |
| Vol'_{3.2} | Volumenfluss durch das zweite Segment 3.2, vom zweiten Volumenfluss-Sensor 6.2 gemessen |
| Vol'₃₀ | Volumenfluss durch die Zuführ-Fluidführungseinheit 30, vom dritten Volumenfluss-Sensor 6.3 gemessen |
| W | Wand, weist die Versorgungsanschluss-Anordnung 13 und die Steckdose 16 auf |

## Patentansprüche

1. Beatmungs-System zur künstlichen Beatmung eines Patienten (Pt), wobei das Beatmungs-System
- ein Beatmungsgerät (100) mit einem Gehäuse (12),
- eine patientenseitige Koppeleinheit (1),
- ein Narkosemitteldosierer-Bauteil (38, 38.1),
- einen Temperatursensor (20, 21, 22) aus einer Temperatursensor-Gruppe,
- einen Datenspeicher (33.1, 33.2) mit einem rechnerauswertbaren funktionalen Zusammenhang (32.1, 32.2) und
- ein signalverarbeitendes Steuergerät (11)
umfasst,
wobei die patientenseitige Koppeleinheit (1) dazu ausgestaltet ist, im und / oder am Körper eines künstlich zu beatmenden Patienten (Pt) angeordnet zu werden,
wobei das Gehäuse (12) des Beatmungsgeräts (100) eine Aufnahme (50, 50.1) umfasst,
wobei das Narkosemitteldosierer-Bauteil (38, 38.1) wenigstens zeitweise in die Aufnahme (50, 50.1) eingesetzt ist,
wobei das Narkosemitteldosierer-Bauteil (38, 38.1) dazu ausgestaltet ist, ein Narkotisierungs-Gasgemisch (Ng, Ng.1) umfassend ein Narkosemittel (Nm) zu erzeugen,
wobei das Beatmungsgerät (100) dazu ausgestaltet ist, ein atembares Gasgemisch (Bg) umfassend Sauerstoff und das erzeugte Narkotisierungs-Gasgemisch (Ng, Ng.1) zur patientenseitigen Koppeleinheit (1) zu fördern,
wobei das Steuergerät (11) dazu ausgestaltet ist, eine Soll-Konzentration (con_{req}) des Narkosemittels (Nm) im zu erzeugenden Narkotisierungs-Gasgemisch (Ng, Ng.1) zu berechnen,
wobei das Steuergerät (11) weiterhin dazu ausgestaltet ist, das Narkosemitteldosierer-Bauteil (38, 38.1) mit dem Ziel anzusteuern, dass das Narkosemitteldosierer-Bauteil (38, 38.1) das Narkotisierungs-Gasgemisch (Ng, Ng.1) mit der berechneten Soll-Konzentration (con_{req}) erzeugt,
wobei die Temperatursensor-Gruppe aus
- einem Umgebungstemperatur-Sensor (20),
- einem narkosemitteldosierer-seitigen Temperatursensor (21) und
- einem beatmungsgeräte-seitigen Temperatursensor (22, 22.1)
besteht,
wobei der Umgebungstemperatur-Sensor (20) dazu ausgestaltet ist, eine Umgebungstemperatur (Temp_{amb}) in einer Umgebung des Beatmungs-Systems zu messen,
wobei der narkosemitteldosierer-seitige Temperatursensor (21) dazu ausgestaltet ist, eine Temperatur (Temp₃₈) an einer Narkosemitteldosierer-Messposition im oder am Narkosemitteldosierer-Bauteil (38, 38.1) zu messen,
wobei der beatmungsgeräte-seitige Temperatursensor (22, 22.1) dazu ausgestaltet ist, eine Temperatur (Temp₅₀) an einer Beatmungsgeräte-Messposition im oder am Beatmungsgerät (100) zu messen,
wobei der funktionale Zusammenhang (32.1, 32.2) eine obere Konzentrations-Schranke (Conₘₐₓ) für die Konzentration des Narkosemittels im Narkotisierungs-Gasgemisch (Ng, Ng.1) in Abhängigkeit von der messbaren Temperatur (Temp_{amb}, Temp₃₈, Temp₅₀) dergestalt spezifiziert,
dass die obere Konzentrations-Schranke (Conₘₐₓ) mit steigender messbarer Temperatur (Temp_{amb}, Temp₃₈, Temp₅₀) größer wird oder mindestens gleichbleibt,
wobei eine messbare Temperatur eine Temperatur ist, die von einem Temperatursensor (20, 21, 22) der Temperatursensor-Gruppe messbar ist,
wobei das Steuergerät (11) weiterhin dazu ausgestaltet ist,
- den funktionalen Zusammenhang (32.1, 32.2) auf einen gemessenen Wert (temp_{amb}, temp₃₈, temp₅₀) einer messbaren Temperatur (Temp_{amb}, Temp₃₈, Temp₅₀) anzuwenden,
- hierdurch einen Wert (conₘₐₓ) für die obere Konzentrations-Schranke (Conₘₐₓ) herzuleiten und
- die Soll-Konzentration (con_{req}) so zu berechnen, dass die Soll-Konzentration (con_{req}) höchstens gleich dem hergeleiteten Wert (conₘₐₓ) für die obere Konzentrations-Schranke (Conₘₐₓ) ist.

2. Beatmungs-System nach Anspruch 1,
**dadurch gekennzeichnet, dass**
das Beatmungs-System den narkosemitteldosierer-seitigen Temperatursensor (21) und den beatmungsgeräte-seitigen Temperatursensor (22, 22.1) umfasst,
wobei der funktionale Zusammenhang (32.1, 32.2) die obere Konzentrations-Schranke (Conₘₐₓ) in Abhängigkeit von der Temperatur (Temp₃₈) an der Narkosemitteldosierer-Messposition und von der Temperatur (Temp₅₀) an der Beatmungsgeräte-Messposition dergestalt spezifiziert,
dass die obere Konzentrations-Schranke (Conₘₐₓ) mit steigender Temperatur (Temp₃₈) an der Narkosemitteldosierer-Messposition bei gleichbleibender Temperatur an der Beatmungsgeräte-Messposition und mit steigender Temperatur (Temp₅₀) an der Beatmungsgeräte-Messposition bei gleichbleibender Temperatur an der Narkosemitteldosierer-Messposition größer wird oder mindestens gleichbleibt,
wobei das Steuergerät (11) dazu ausgestaltet ist, bei der Herleitung des Werts (conₘₐₓ) für die obere Konzentrations-Schranke (Conₘₐₓ) den funktionalen Zusammenhang (32.1, 32.2) auf den gemessenen Wert (temp₃₈) der Temperatur (Temp₃₈) an der Narkosemitteldosierer-Messposition und auf den gemessenen Wert (temp₅₀) der Temperatur (Temp₅₀) an der Beatmungsgeräte-Messposition anzuwenden.

3. Beatmungs-System nach Anspruch 2,
**dadurch gekennzeichnet, dass**
die Narkosemitteldosierer-Messposition in einem thermischen Kontakt mit einer Oberfläche des Narkosemitteldosierer-Bauteils (38, 38.1), wobei die Oberfläche bei eingesetztem Narkosemitteldosierer-Bauteil (38, 38.1) zur Aufnahme (50, 50.1) hin zeigt, steht und / oder
die Beatmungsgeräte-Messposition in einem thermischen Kontakt mit einer Oberfläche der Aufnahme (50, 50.1), wobei die Oberfläche bei eingesetztem Narkosemitteldosierer-Bauteil (38, 38.1) zum Narkosemitteldosierer-Bauteil (38, 38.1) hin zeigt.

4. Beatmungs-System nach einem der vorhergehenden Ansprüche,
**dadurch gekennzeichnet, dass**
das Beatmungs-System einen Narkosemittel-Sensor (27) umfasst,
wobei der Narkosemittel-Sensor (27) dazu ausgestaltet ist, die Konzentration des Narkosemittels (Nm, Nm.1) im erzeugten Narkotisierungs-Gasgemisch (Ng, Ng.1) zu messen, und
wobei das Steuergerät (11) dazu ausgestaltet ist, unter Verwendung eines Signals des Narkosemittel-Sensors (27) die Konzentration des Narkosemittels (Nm, Nm.1) im erzeugten Narkotisierungs-Gasgemisch (Ng, Ng.1) mit dem Regelungsziel zu regeln, dass die tatsächliche Narkosemittel-Konzentration gleich der berechneten Soll-Konzentration (con_{req}) ist.

5. Beatmungs-System nach Anspruch 4,
**dadurch gekennzeichnet, dass**
das Beatmungs-System den beatmungsgeräte-seitigen Temperatursensor (22, 22.1) umfasst und
die Beatmungsgeräte-Messposition in einem thermischen Kontakt mit dem Narkosemittel-Sensor (27) steht.

6. Beatmungs-System nach einem der vorhergehenden Ansprüche,
**dadurch gekennzeichnet, dass**
das Beatmungsgerät (100) so ausgestaltet ist, dass sich das Narkosemitteldosierer-Bauteil (38, 38.1) in die Aufnahme (50, 50.1) einsetzen und wieder aus der Aufnahme (50, 50.1) entnehmen lässt, und
das Beatmungs-System
- den Umgebungstemperatur-Sensor (20),
- einen Eingesetzt-Sensor (51) und
- eine Systemuhr (52)
umfasst,
wobei der Eingesetzt-Sensor (51) dazu ausgestaltet ist zu detektieren, ob in die Aufnahme (50, 50.1) das Narkosemitteldosierer-Bauteil (38, 38.1) eingesetzt ist oder nicht,
wobei das Steuergerät (11) dazu ausgestaltet ist, bei eingesetztem Narkosemitteldosierer-Bauteil (38, 38.1) abhängig von einem Signal des Eingesetzt-Sensors (51) und einem Signal der Systemuhr (52) die Eingesetzt-Zeitdauer (ΔT) zu messen, die seit dem letzten Einsetzen des Narkosemitteldosierer-Bauteils (38, 38.1) verstrichen ist,
wobei der funktionale Zusammenhang (32.1, 32.2) die obere Konzentrations-Schranke (Conₘₐₓ) in Abhängigkeit von der Umgebungstemperatur (Temp_{amb}) und zusätzlich in Abhängigkeit von der Eingesetzt-Zeitdauer (ΔT) dergestalt spezifiziert, dass die obere Konzentrations-Schranke (Conₘₐₓ) bei gleichbleibender Umgebungstemperatur (Temp_{amb}) mit steigender Eingesetzt-Zeitdauer (ΔT) größer wird oder mindestens gleichbleibt, und
wobei das Steuergerät (11) dazu ausgestaltet ist, bei der Herleitung des Werts (conₘₐₓ) für die obere Konzentrations-Schranke (Conₘₐₓ) den funktionalen Zusammenhang (32.1, 32.2) auf den gemessenen Wert (temp_{amb}) der Umgebungstemperatur (Temp_{amb}) und zusätzlich auf den gemessenen Wert (Δt) der Eingesetzt-Zeitdauer (ΔT) anzuwenden.

7. Beatmungs-System nach einem der vorhergehenden Ansprüche,
**dadurch gekennzeichnet, dass**
eine Menge mit mindestens zwei verschiedenen möglichen Narkosemitteln (Nm, Nm.1) vorgegeben ist und
der funktionale Zusammenhang für jedes mögliche Narkosemittel (Nm, Nm.1) der Menge jeweils einen funktionalen Einzel-Zusammenhang {32.1[Nm], 32.1[Nm.1], 32.2[Nm], 32.2[Nm.1]} umfasst,
wobei jeder funktionale Einzel-Zusammenhang {32.1[Nm], 32.1[Nm.1], 32.2[Nm], 32.2[Nm.1]} die obere Konzentrations-Schranke (Conₘₐₓ) in Abhängigkeit von der messbaren Temperatur (Temp_{amb}, Temp₃₈, Temp₅₀), dergestalt spezifiziert,
dass die obere Konzentrations-Schranke (Conₘₐₓ) mit steigender messbarer Temperatur (Temp_{amb}, Temp₃₈, Temp₅₀ ) größer wird oder mindestens gleichbleibt,
wobei das Narkosemitteldosierer-Bauteil (38, 38.1) dazu ausgestaltet ist, ein Narkotisierungs-Gasgemisch (Ng, Ng.1) zu erzeugen, welches eines der möglichen Narkosemittel (Nm, Nm.1) umfasst,
wobei das Steuergerät (11) dazu ausgestaltet ist, für jedes mögliche Narkosemittel (Nm, Nm.1) jeweils eine Soll-Konzentration (con_{req}) zu berechnen und hierfür
- den funktionalen Einzel-Zusammenhang {32.1[Nm], 32.1[Nm.1], 32.2[Nm], 32.2[Nm.1]} für dieses Narkosemittel (Nm, Nm.1) auf einen gemessenen Wert (temp_{amb}, temp₃₈, temp₅₀) einer messbaren Temperatur (Temp_{amb}, Temp₃₈, Temp₅₀) anzuwenden,
- hierdurch für dieses Narkosemittel (Nm, Nm.1) einen Wert (conₘₐₓ) für die obere Konzentrations-Schranke (Conₘₐₓ) herzuleiten und
- die Soll-Konzentration (con_{req}) so zu berechnen, dass die Soll-Konzentration höchstens gleich dem hergeleiteten Wert (conₘₐₓ) für die obere Konzentrations-Schranke (Conₘₐₓ) ist.

8. Beatmungs-System nach Anspruch 7,
**dadurch gekennzeichnet, dass**
das Steuergerät (11) dazu ausgestaltet ist,
- dann, wenn das Narkosemitteldosierer-Bauteil (38, 38.1) in die Aufnahme (50, 50.1) eingesetzt ist,
zu erfassen, welches Narkosemittel (Nm, Nm.1) das Narkotisierungs-Gasgemisch (Ng, Ng.1), das vom Narkosemitteldosierer-Bauteil (38, 38.1) generiert wird, umfasst,
- den funktionalen Einzel-Zusammenhang {32.1[Nm], 32.1[Nm.1], 32.2[Nm], 32.2[Nm.1]} für das erfasste Narkosemittel (Nm, Nm.1) auszuwählen und
- die Soll-Konzentration (con_{req}) unter Verwendung des ausgewählten funktionalen Einzel-Zusammenhangs {32.1[Nm], 32.1[Nm.1], 32.2[Nm], 32.2[Nm.1]} zu berechnen.

9. Beatmungs-System nach einem der vorhergehenden Ansprüche,
**dadurch gekennzeichnet, dass**
das Steuergerät (11) dazu ausgestaltet ist, eine Vorgabe zu erfassen,
wobei die erfasste Vorgabe eine Vorgabe-Konzentration und / oder einen Vorgabe-Volumenfluss und / oder einen Vorgabe-Massefluss des Narkosemittels (Nm, Nm.1) im zu erzeugenden Narkotisierungs-Gasgemisch (Ng, Ng.1) spezifiziert,
wobei das Steuergerät (11) weiterhin dazu ausgestaltet ist, die Soll-Konzentration (con_{req}) des Narkosemittels (Nm, Nm.1) abhängig von der erfassten Vorgabe herzuleiten und dadurch zu berechnen.

10. Ansteuer-Anordnung zum Ansteuern einer Beatmungs-Anordnung,
wobei die Beatmungs-Anordnung zur künstlichen Beatmung eines Patienten (Pt) ausgestaltet ist und
- ein Beatmungsgerät (100) mit einem Gehäuse (12),
- eine patientenseitige Koppeleinheit (1) und
- ein Narkosemitteldosierer-Bauteil (38, 38.1)
umfasst,
wobei die patientenseitige Koppeleinheit (1) dazu ausgestaltet ist, im und / oder am Körper eines künstlich zu beatmenden Patienten (Pt) angeordnet zu werden,
wobei das Gehäuse (12) des Beatmungsgeräts (100) eine Aufnahme (50, 50.1) umfasst,
wobei das Narkosemitteldosierer-Bauteil (38, 38.1) wenigstens zeitweise in die Aufnahme (50, 50.1) eingesetzt ist,
wobei das Narkosemitteldosierer-Bauteil (38, 38.1) dazu ausgestaltet ist, ein Narkotisierungs-Gasgemisch (Ng, Ng.1) umfassend ein Narkosemittel (Nm) zu erzeugen, und
wobei das Beatmungsgerät (100) dazu ausgestaltet ist, ein atembares Gasgemisch (Bg) umfassend Sauerstoff und das erzeugte Narkotisierungs-Gasgemisch (Ng, Ng.1) zur patientenseitigen Koppeleinheit (1) zu fördern,
wobei die Ansteuer-Anordnung
- einen Temperatursensor (20, 21, 22) aus einer Temperatursensor-Gruppe,
- einen Datenspeicher (33.1, 33.2) mit einem rechnerauswertbaren funktionalen Zusammenhang (32.1, 32.2) und
- ein signalverarbeitendes Steuergerät (11)
umfasst,
wobei die Temperatursensor-Gruppe aus
- einem Umgebungstemperatur-Sensor (20),
- einem narkosemitteldosierer-seitigen Temperatursensor (21) und
- einem beatmungsgeräte-seitigen Temperatursensor (22, 22.1)
besteht,
wobei der Umgebungstemperatur-Sensor (20) dazu ausgestaltet ist, eine Umgebungstemperatur (Temp_{amb}) in einer Umgebung der Beatmungs-Anordnung zu messen,
wobei der narkosemitteldosierer-seitige Temperatursensor (21) dazu ausgestaltet ist, eine Temperatur (Temp₃₈) an einer Narkosemitteldosierer-Messposition im oder am Narkosemitteldosierer-Bauteil (38, 38.1) zu messen,
wobei der beatmungsgeräte-seitige Temperatursensor (22, 22.1) dazu ausgestaltet ist, eine Temperatur (Temp₅₀) an einer Beatmungsgeräte-Messposition im oder am Beatmungsgerät (100) zu messen,
wobei der funktionale Zusammenhang (32.1, 32.2) eine obere Konzentrations-Schranke (Conₘₐₓ) für die Konzentration des Narkosemittels im Narkotisierungs-Gasgemisch (Ng, Ng.1) in Abhängigkeit von einer messbaren Temperatur (Temp_{amb}, Temp₃₈, Temp₅₀) dergestalt spezifiziert,
dass die obere Konzentrations-Schranke (Conₘₐₓ) mit steigender messbarer Temperatur (Temp_{amb}, Temp₃₈, Temp₅₀) größer wird oder mindestens gleichbleibt,
wobei eine messbare Temperatur eine Temperatur ist, die von einem Temperatursensor (20, 21, 22) der Temperatursensor-Gruppe messbar ist,
wobei das Steuergerät (11) dazu ausgestaltet ist, eine Soll-Konzentration (con_{req}) des Narkosemittels (Nm) im zu erzeugenden Narkotisierungs-Gasgemisch (Ng, Ng.1) zu berechnen,
wobei das Steuergerät (11) weiterhin dazu ausgestaltet ist, das Narkosemitteldosierer-Bauteil (38, 38.1) mit dem Ziel anzusteuern, dass das Narkosemitteldosierer-Bauteil (38, 38.1) das Narkotisierungs-Gasgemisch (Ng, Ng.1) mit der berechneten Soll-Konzentration (con_{req}) bereitstellt,
wobei das Steuergerät (11) weiterhin dazu ausgestaltet ist,
- den funktionalen Zusammenhang (32.1, 32.2) auf einen gemessenen Wert (temp_{amb}, temp₃₈, temp₅₀) einer messbaren Temperatur (Temp_{amb}, Temp₃₈, Temp₅₀) anzuwenden,
- hierdurch einen Wert (conₘₐₓ) für die obere Konzentrations-Schranke (Conₘₐₓ) herzuleiten und
- die Soll-Konzentration (con_{req}) so zu berechnen, dass die Soll-Konzentration (con_{req}) höchstens gleich dem hergeleiteten Wert (conₘₐₓ) für die obere Konzentrations-Schranke (Conₘₐₓ) ist.

11. Ansteuer-Verfahren zum Ansteuern einer Beatmungs-Anordnung,
wobei die Beatmungs-Anordnung zur künstlichen Beatmung eines Patienten (Pt) ausgestaltet ist und
- ein Beatmungsgerät (100) mit einem Gehäuse (12),
- eine patientenseitige Koppeleinheit (1) und
- ein Narkosemitteldosierer-Bauteil (38, 38.1),
umfasst,
wobei die patientenseitige Koppeleinheit (1) dazu ausgestaltet ist, im und / oder am Körper eines künstlich zu beatmenden Patienten (Pt) angeordnet zu werden,
wobei das Gehäuse (12) des Beatmungsgeräts (100) eine Aufnahme (50, 50.1) umfasst,
wobei das Narkosemitteldosierer-Bauteil (38, 38.1) wenigstens zeitweise in die Aufnahme (50, 50.1) eingesetzt ist,
wobei das Narkosemitteldosierer-Bauteil (38, 38.1) dazu ausgestaltet ist, ein Narkotisierungs-Gasgemisch (Ng, Ng.1) umfassend ein Narkosemittel (Nm) zu erzeugen, und
wobei das Beatmungsgerät (100) dazu ausgestaltet ist, ein atembares Gasgemisch (Bg) umfassend Sauerstoff und das erzeugte Narkotisierungs-Gasgemisch (Ng, Ng.1) zur patientenseitigen Koppeleinheit (1) zu fördern, wobei das Ansteuer-Verfahren unter Verwendung
- eines Temperatursensors (20, 21, 22) aus einer Temperatursensor-Gruppe,
- eines Datenspeichers (33.1, 33.2) mit einem rechnerauswertbaren funktionalen Zusammenhang (32.1, 32.2) und
- eines signalverarbeitenden Steuergeräts (11)
durchgeführt wird,
wobei die Temperatursensor-Gruppe aus
- einem Umgebungstemperatur-Sensor (20),
- einem narkosemitteldosierer-seitigen Temperatursensor (21) und
- einem beatmungsgeräte-seitigen Temperatursensor (22, 22.1)
besteht,
wobei der funktionale Zusammenhang (32.1, 32.2) eine obere Konzentrations-Schranke (Conₘₐₓ) für die Konzentration des Narkosemittels im Narkotisierungs-Gasgemisch (Ng, Ng.1) in Abhängigkeit von einer messbaren Temperatur (Temp_{amb}, Temp₃₈, Temp₅₀) dergestalt spezifiziert,
dass die obere Konzentrations-Schranke (Conₘₐₓ) mit steigender messbarer Temperatur (Temp_{amb}, Temp₃₈, Temp₅₀) größer wird oder mindestens gleichbleibt,
wobei die messbare Temperatur (Temp_{amb}, Temp₃₈, Temp₅₀)
- eine Umgebungstemperatur (Temp_{amb}) in einer Umgebung der Beatmungs-Anordnung und / oder
- eine Temperatur (Temp₃₈) an einer Narkosemitteldosierer-Messposition im oder am Narkosemitteldosierer-Bauteil (38, 38.1) und / oder
- eine Temperatur (Temp₅₀) an einer Beatmungsgeräte-Messposition im oder am Beatmungsgerät (100)
ist,
wobei das Ansteuer-Verfahren die Schritte umfasst, dass
- ein Temperatursensor (20, 21, 22) der Temperatursensor-Gruppe eine messbare Temperatur (Temp_{amb}, Temp₃₈, Temp₅₀) misst,
- das Steuergerät (11) eine Soll-Konzentration (con_{req}) des Narkosemittels (Nm, Nm.1) im zu erzeugenden Narkotisierungs-Gasgemisch (Ng, Ng.1) berechnet und
- das Narkosemitteldosierer-Bauteil (38, 38.1) mit dem Ziel ansteuert, dass das Narkosemitteldosierer-Bauteil (38, 38.1) das Narkotisierungs-Gasgemisch (Ng, Ng.1) mit der berechneten Soll-Konzentration (con_{req}) bereitstellt, und
wobei der Schritt, dass das Steuergerät (11) die Soll-Konzentration (con_{req}) berechnet, die Schritte umfasst, dass das Steuergerät (11)
- den funktionalen Zusammenhang (32.1, 32.2) auf einen gemessenen Wert (temp_{amb}, temp₃₈, temp₅₀) einer messbaren Temperatur (Temp_{amb}, Temp₃₈, Temp₅₀) anwendet,
- hierdurch einen Wert (conₘₐₓ) für die obere Konzentrations-Schranke (Conₘₐₓ) herleitet und
- die Soll-Konzentration (con_{req}) so berechnet, dass die Soll-Konzentration (con_{req}) höchstens gleich dem hergeleiteten Wert (conₘₐₓ) für die obere Konzentrations-Schranke (Conₘₐₓ) ist.

12. Beatmungs-Verfahren zur künstlichen Beatmung eines Patienten (Pt),
wobei das Beatmungs-Verfahren unter Verwendung einer Beatmungs-Anordnung durchgeführt wird,
wobei die verwendete Beatmungs-Anordnung
- ein Beatmungsgerät (100) mit einem Gehäuse (12),
- eine patientenseitige Koppeleinheit (1)
- ein Narkosemitteldosierer-Bauteil (38, 38.1),
- einen Temperatursensor (20, 21, 22) aus einer Temperatursensor-Gruppe,
- einen Datenspeicher (33.1, 33.2) mit einem rechnerauswertbaren funktionalen Zusammenhang (32.1, 32.2) und
- ein signalverarbeitendes Steuergerät (11)
umfasst,
wobei das Gehäuse (12) des Beatmungsgeräts (100) eine Aufnahme (50, 50.1) umfasst,
wobei der funktionale Zusammenhang (32.1, 32.2) eine obere Konzentrations-Schranke (Conₘₐₓ) für die Konzentration des Narkosemittels im Narkotisierungs-Gasgemisch (Ng, Ng.1) in Abhängigkeit von einer messbaren Temperatur (Temp_{amb}, Temp₃₈, Temp₅₀) dergestalt spezifiziert,
dass die obere Konzentrations-Schranke (Conₘₐₓ) mit steigender messbarer Temperatur (Temp_{amb}, Temp₃₈, Temp₅₀) größer wird oder mindestens gleichbleibt,
wobei die messbare Temperatur (Temp_{amb}, Temp₃₈, Temp₅₀)
- eine Umgebungstemperatur (Temp_{amb}) in einer Umgebung der Beatmungs-Anordnung und / oder
- eine Temperatur (Temp₃₈) an einer Narkosemitteldosierer-Messposition im oder am Narkosemitteldosierer-Bauteil (38, 38.1) und / oder
- eine Temperatur (Temp₅₀) an einer Beatmungsgeräte-Messposition im oder am Beatmungsgerät (100)
ist,
wobei das Beatmungs-Verfahren durchgeführt wird, während
- die patientenseitigen Koppeleinheit (1) im und / oder am Körper des Patienten (Pt) angeordnet ist und
- in die Aufnahme (50, 50.1) das Narkosemitteldosierer-Bauteil (38, 38.1) eingesetzt ist,
wobei das Verfahren die Schritte umfasst, dass
- ein Temperatursensor (20, 21, 22) der Temperatursensor-Gruppe eine messbare Temperatur (Temp_{amb}, Temp₃₈, Temp₅₀) misst,
- das eingesetzte Narkosemitteldosierer-Bauteil (38, 38.1) ein Narkotisierungs-Gasgemisch (Ng, Ng.1) umfassend ein Narkosemittel (Nm) erzeugt und
- das Beatmungsgerät (100) ein atembares Gasgemisch (Bg) umfassend das erzeugte Narkotisierungs-Gasgemisch (Ng, Ng.1) zur patientenseitigen Koppeleinheit (1) fördert,
wobei das Verfahren die weiteren Schritte umfasst, dass das Steuergerät (11)
- eine Soll-Konzentration (con_{req}) des Narkosemittels (Nm, Nm.1) im zu erzeugenden Narkotisierungs-Gasgemisch (Ng, Ng.1) berechnet und
- das Narkosemitteldosierer-Bauteil (38, 38.1) mit dem Ziel ansteuert, dass das Narkosemitteldosierer-Bauteil (38, 38.1) das Narkotisierungs-Gasgemisch (Ng, Ng.1) mit der berechneten Soll-Konzentration bereitstellt, und
wobei der Schritt, dass das Steuergerät (11) die Soll-Konzentration (con_{req}) berechnet, die Schritte umfasst, dass das Steuergerät (11)
- den funktionalen Zusammenhang (32.1, 32.2) auf einen gemessenen Wert (temp_{amb}, temp₃₈, temp₅₀) einer messbaren Temperatur (Temp_{amb}, Temp₃₈, Temp₅₀) anwendet,
- hierdurch einen Wert (conₘₐₓ) für die obere Konzentrations-Schranke (Conₘₐₓ) herleitet und
- die Soll-Konzentration (con_{req}) so berechnet, dass die Soll-Konzentration (con_{req}) höchstens gleich dem hergeleiteten Wert (conₘₐₓ) für die obere Konzentrations-Schranke (Conₘₐₓ) ist.

## Geänderte Patentansprüche

### Geänderte Patentansprüche gemäss Regel 137(2) EPÜ.

1. Beatmungs-System zur künstlichen Beatmung eines Patienten (Pt), wobei das Beatmungs-System
- ein Beatmungsgerät (100) mit einem Gehäuse (12),
- eine patientenseitige Koppeleinheit (1),
- ein Narkosemitteldosierer-Bauteil (38, 38.1),
- einen Temperatursensor (20, 21, 22) und
- ein signalverarbeitendes Steuergerät (11)
umfasst,
wobei die patientenseitige Koppeleinheit (1) dazu ausgestaltet ist, im und / oder am Körper eines künstlich zu beatmenden Patienten (Pt) angeordnet zu werden,
wobei das Gehäuse (12) des Beatmungsgeräts (100) eine Aufnahme (50, 50.1) umfasst,
wobei das Narkosemitteldosierer-Bauteil (38, 38.1) wenigstens zeitweise in die Aufnahme (50, 50.1) eingesetzt ist,
wobei das Narkosemitteldosierer-Bauteil (38, 38.1) dazu ausgestaltet ist, ein Narkotisierungs-Gasgemisch (Ng, Ng.1) umfassend ein Narkosemittel (Nm) zu erzeugen,
wobei das Beatmungsgerät (100) dazu ausgestaltet ist, ein atembares Gasgemisch (Bg) umfassend Sauerstoff und das erzeugte Narkotisierungs-Gasgemisch (Ng, Ng.1) zur patientenseitigen Koppeleinheit (1) zu fördern,
wobei das Steuergerät (11) dazu ausgestaltet ist, eine Soll-Konzentration (con_{req}) des Narkosemittels (Nm) im zu erzeugenden Narkotisierungs-Gasgemisch (Ng, Ng.1) zu berechnen, und
wobei das Steuergerät (11) weiterhin dazu ausgestaltet ist, das Narkosemitteldosierer-Bauteil (38, 38.1) mit dem Ziel anzusteuern, dass das Narkosemitteldosierer-Bauteil (38, 38.1) das Narkotisierungs-Gasgemisch (Ng, Ng.1) mit der berechneten Soll-Konzentration (con_{req}) erzeugt,
**dadurch gekennzeichnet, dass**
das Beatmungs-System einen Datenspeicher (33.1, 33.2) mit einem rechnerauswertbaren funktionalen Zusammenhang (32.1, 32.2) umfasst und der Temperatursensor (20, 21, 22) aus einer Temperatursensor-Gruppe ist, wobei die Temperatursensor-Gruppe aus
- einem Umgebungstemperatur-Sensor (20),
- einem narkosemitteldosierer-seitigen Temperatursensor (21) und
- einem beatmungsgeräte-seitigen Temperatursensor (22, 22.1)
besteht,
wobei der Umgebungstemperatur-Sensor (20) dazu ausgestaltet ist, eine Umgebungstemperatur (Temp_{amb}) in einer Umgebung des Beatmungs-Systems zu messen,
wobei der narkosemitteldosierer-seitige Temperatursensor (21) dazu ausgestaltet ist, eine Temperatur (Temp₃₈) an einer Narkosemitteldosierer-Messposition im oder am Narkosemitteldosierer-Bauteil (38, 38.1) zu messen,
wobei der beatmungsgeräte-seitige Temperatursensor (22, 22.1) dazu ausgestaltet ist, eine Temperatur (Temp₅₀) an einer Beatmungsgeräte-Messposition im oder am Beatmungsgerät (100) zu messen,
wobei der funktionale Zusammenhang (32.1, 32.2) eine obere Konzentrations-Schranke (Conₘₐₓ) für die Konzentration des Narkosemittels im Narkotisierungs-Gasgemisch (Ng, Ng.1) in Abhängigkeit von einer messbaren Temperatur (Temp_{amb}, Temp₃₈, Temp₅₀) dergestalt spezifiziert,
dass die obere Konzentrations-Schranke (Conₘₐₓ) mit steigender messbarer Temperatur (Temp_{amb}, Temp₃₈, Temp₅₀) größer wird oder mindestens gleichbleibt,
wobei die messbare Temperatur eine Temperatur ist, die von einem Temperatursensor (20, 21, 22) der Temperatursensor-Gruppe messbar ist, wobei das Steuergerät (11) weiterhin dazu ausgestaltet ist,
- den funktionalen Zusammenhang (32.1, 32.2) auf einen gemessenen Wert (temp_{amb}, temp₃₈, temp₅₀) einer messbaren Temperatur (Temp_{amb}, Temp₃₈, Temp₅₀) anzuwenden,
- hierdurch einen Wert (conₘₐₓ) für die obere Konzentrations-Schranke (Conₘₐₓ) herzuleiten und
- die Soll-Konzentration (con_{req}) so zu berechnen, dass die Soll-Konzentration (con_{req}) höchstens gleich dem hergeleiteten Wert (conₘₐₓ) für die obere Konzentrations-Schranke (Conₘₐₓ) ist.

2. Beatmungs-System nach Anspruch 1,
**dadurch gekennzeichnet, dass**
das Beatmungs-System den narkosemitteldosierer-seitigen Temperatursensor (21) und den beatmungsgeräte-seitigen Temperatursensor (22, 22.1) umfasst,
wobei der funktionale Zusammenhang (32.1, 32.2) die obere Konzentrations-Schranke (Conₘₐₓ) in Abhängigkeit von der Temperatur (Temp₃₈) an der Narkosemitteldosierer-Messposition und von der Temperatur (Temp₅₀) an der Beatmungsgeräte-Messposition dergestalt spezifiziert,
dass die obere Konzentrations-Schranke (Conₘₐₓ) mit steigender Temperatur (Temp₃₈) an der Narkosemitteldosierer-Messposition bei gleichbleibender Temperatur an der Beatmungsgeräte-Messposition und mit steigender Temperatur (Temp₅₀) an der Beatmungsgeräte-Messposition bei gleichbleibender Temperatur an der Narkosemitteldosierer-Messposition größer wird oder mindestens gleichbleibt,
wobei das Steuergerät (11) dazu ausgestaltet ist, bei der Herleitung des Werts (conₘₐₓ) für die obere Konzentrations-Schranke (Conₘₐₓ) den funktionalen Zusammenhang (32.1, 32.2) auf den gemessenen Wert (temp₃₈) der Temperatur (Temp₃₈) an der Narkosemitteldosierer-Messposition und auf den gemessenen Wert (temp₅₀) der Temperatur (Temp₅₀) an der Beatmungsgeräte-Messposition anzuwenden.

3. Beatmungs-System nach Anspruch 2,
**dadurch gekennzeichnet, dass**
die Narkosemitteldosierer-Messposition in einem thermischen Kontakt mit einer Oberfläche des Narkosemitteldosierer-Bauteils (38, 38.1), wobei die Oberfläche bei eingesetztem Narkosemitteldosierer-Bauteil (38, 38.1) zur Aufnahme (50, 50.1) hin zeigt, steht und / oder
die Beatmungsgeräte-Messposition in einem thermischen Kontakt mit einer Oberfläche der Aufnahme (50, 50.1), wobei die Oberfläche bei eingesetztem Narkosemitteldosierer-Bauteil (38, 38.1) zum Narkosemitteldosierer-Bauteil (38, 38.1) hin zeigt.

4. Beatmungs-System nach einem der vorhergehenden Ansprüche,
**dadurch gekennzeichnet, dass**
das Beatmungs-System einen Narkosemittel-Sensor (27) umfasst, wobei der Narkosemittel-Sensor (27) dazu ausgestaltet ist, die Konzentration des Narkosemittels (Nm, Nm.1) im erzeugten Narkotisierungs-Gasgemisch (Ng, Ng.1) zu messen, und
wobei das Steuergerät (11) dazu ausgestaltet ist, unter Verwendung eines Signals des Narkosemittel-Sensors (27) die Konzentration des Narkosemittels (Nm, Nm.1) im erzeugten Narkotisierungs-Gasgemisch (Ng, Ng.1) mit dem Regelungsziel zu regeln, dass die tatsächliche Narkosemittel-Konzentration gleich der berechneten Soll-Konzentration (con_{req}) ist.

5. Beatmungs-System nach Anspruch 4,
**dadurch gekennzeichnet, dass**
das Beatmungs-System den beatmungsgeräte-seitigen Temperatursensor (22, 22.1) umfasst und
die Beatmungsgeräte-Messposition in einem thermischen Kontakt mit dem Narkosemittel-Sensor (27) steht.

6. Beatmungs-System nach einem der vorhergehenden Ansprüche,
**dadurch gekennzeichnet, dass**
das Beatmungsgerät (100) so ausgestaltet ist, dass sich das Narkosemitteldosierer-Bauteil (38, 38.1) in die Aufnahme (50, 50.1) einsetzen und wieder aus der Aufnahme (50, 50.1) entnehmen lässt, und
das Beatmungs-System
- den Umgebungstemperatur-Sensor (20),
- einen Eingesetzt-Sensor (51) und
- eine Systemuhr (52)
umfasst,
wobei der Eingesetzt-Sensor (51) dazu ausgestaltet ist zu detektieren, ob in die Aufnahme (50, 50.1) das Narkosemitteldosierer-Bauteil (38, 38.1) eingesetzt ist oder nicht,
wobei das Steuergerät (11) dazu ausgestaltet ist, bei eingesetztem Narkosemitteldosierer-Bauteil (38, 38.1) abhängig von einem Signal des Eingesetzt-Sensors (51) und einem Signal der Systemuhr (52) die Eingesetzt-Zeitdauer (ΔT) zu messen, die seit dem letzten Einsetzen des Narkosemitteldosierer-Bauteils (38, 38.1) verstrichen ist,
wobei der funktionale Zusammenhang (32.1, 32.2) die obere Konzentrations-Schranke (Conₘₐₓ) in Abhängigkeit von der Umgebungstemperatur (Temp_{amb}) und zusätzlich in Abhängigkeit von der Eingesetzt-Zeitdauer (ΔT) dergestalt spezifiziert, dass die obere Konzentrations-Schranke (Conₘₐₓ) bei gleichbleibender Umgebungstemperatur (Temp_{amb}) mit steigender Eingesetzt-Zeitdauer (ΔT) größer wird oder mindestens gleichbleibt, und wobei das Steuergerät (11) dazu ausgestaltet ist, bei der Herleitung des Werts (conₘₐₓ) für die obere Konzentrations-Schranke (Conₘₐₓ) den funktionalen Zusammenhang (32.1, 32.2) auf den gemessenen Wert (temp_{amb}) der Umgebungstemperatur (Temp_{amb}) und zusätzlich auf den gemessenen Wert (Δt) der Eingesetzt-Zeitdauer (ΔT) anzuwenden.

7. Beatmungs-System nach einem der vorhergehenden Ansprüche,
**dadurch gekennzeichnet, dass**
eine Menge mit mindestens zwei verschiedenen möglichen Narkosemitteln (Nm, Nm.1) vorgegeben ist und
der funktionale Zusammenhang für jedes mögliche Narkosemittel (Nm, Nm.1) der Menge jeweils einen funktionalen Einzel-Zusammenhang {32.1[Nm], 32.1[Nm.1], 32.2[Nm], 32.2[Nm.1]} umfasst,
wobei jeder funktionale Einzel-Zusammenhang {32.1[Nm], 32.1[Nm.1], 32.2[Nm], 32.2[Nm.1]} die obere Konzentrations-Schranke (Conₘₐₓ) in Abhängigkeit von der messbaren Temperatur (Temp_{amb}, Temp₃₈, Temp₅₀), dergestalt spezifiziert,
dass die obere Konzentrations-Schranke (Conₘₐₓ) mit steigender messbarer Temperatur (Temp_{amb}, Temp₃₈, Temp₅₀ ) größer wird oder mindestens gleichbleibt,
wobei das Narkosemitteldosierer-Bauteil (38, 38.1) dazu ausgestaltet ist, ein Narkotisierungs-Gasgemisch (Ng, Ng.1) zu erzeugen, welches eines der möglichen Narkosemittel (Nm, Nm.1) umfasst,
wobei das Steuergerät (11) dazu ausgestaltet ist, für jedes mögliche Narkosemittel (Nm, Nm.1) jeweils eine Soll-Konzentration (con_{req}) zu berechnen und hierfür
- den funktionalen Einzel-Zusammenhang {32.1[Nm], 32.1[Nm.1], 32.2[Nm], 32.2[Nm.1]} für dieses Narkosemittel (Nm, Nm.1) auf einen gemessenen Wert (temp_{amb}, temp₃₈, temp₅₀) einer messbaren Temperatur (Temp_{amb}, Temp₃₈, Temp₅₀) anzuwenden,
- hierdurch für dieses Narkosemittel (Nm, Nm.1) einen Wert (conₘₐₓ) für die obere Konzentrations-Schranke (Conₘₐₓ) herzuleiten und
- die Soll-Konzentration (con_{req}) so zu berechnen, dass die Soll-Konzentration höchstens gleich dem hergeleiteten Wert (conₘₐₓ) für die obere Konzentrations-Schranke (Conₘₐₓ) ist.

8. Beatmungs-System nach Anspruch 7,
**dadurch gekennzeichnet, dass**
das Steuergerät (11) dazu ausgestaltet ist,
- dann, wenn das Narkosemitteldosierer-Bauteil (38, 38.1) in die Aufnahme (50, 50.1) eingesetzt ist,
zu erfassen, welches Narkosemittel (Nm, Nm.1) das Narkotisierungs-Gasgemisch (Ng, Ng.1), das vom Narkosemitteldosierer-Bauteil (38, 38.1) generiert wird, umfasst,
- den funktionalen Einzel-Zusammenhang {32.1[Nm], 32.1[Nm.1], 32.2[Nm], 32.2[Nm.1]} für das erfasste Narkosemittel (Nm, Nm.1) auszuwählen und
- die Soll-Konzentration (con_{req}) unter Verwendung des ausgewählten funktionalen Einzel-Zusammenhangs {32.1[Nm], 32.1[Nm.1], 32.2[Nm], 32.2[Nm.1]} zu berechnen.

9. Beatmungs-System nach einem der vorhergehenden Ansprüche,
**dadurch gekennzeichnet, dass**
das Steuergerät (11) dazu ausgestaltet ist, eine Vorgabe zu erfassen, wobei die erfasste Vorgabe eine Vorgabe-Konzentration und / oder einen Vorgabe-Volumenfluss und / oder einen Vorgabe-Massefluss des Narkosemittels (Nm, Nm.1) im zu erzeugenden Narkotisierungs-Gasgemisch (Ng, Ng.1) spezifiziert,
wobei das Steuergerät (11) weiterhin dazu ausgestaltet ist, die Soll-Konzentration (con_{req}) des Narkosemittels (Nm, Nm.1) abhängig von der erfassten Vorgabe herzuleiten und dadurch zu berechnen.

10. Ansteuer-Anordnung zum Ansteuern einer Beatmungs-Anordnung,
wobei die Beatmungs-Anordnung zur künstlichen Beatmung eines Patienten (Pt) ausgestaltet ist und
- ein Beatmungsgerät (100) mit einem Gehäuse (12),
- eine patientenseitige Koppeleinheit (1) und
- ein Narkosemitteldosierer-Bauteil (38, 38.1)
umfasst,
wobei die patientenseitige Koppeleinheit (1) dazu ausgestaltet ist, im und / oder am Körper eines künstlich zu beatmenden Patienten (Pt) angeordnet zu werden,
wobei das Gehäuse (12) des Beatmungsgeräts (100) eine Aufnahme (50, 50.1) umfasst,
wobei das Narkosemitteldosierer-Bauteil (38, 38.1) wenigstens zeitweise in die Aufnahme (50, 50.1) eingesetzt ist,
wobei das Narkosemitteldosierer-Bauteil (38, 38.1) dazu ausgestaltet ist, ein Narkotisierungs-Gasgemisch (Ng, Ng.1) umfassend ein Narkosemittel (Nm) zu erzeugen, und
wobei das Beatmungsgerät (100) dazu ausgestaltet ist, ein atembares Gasgemisch (Bg) umfassend Sauerstoff und das erzeugte Narkotisierungs-Gasgemisch (Ng, Ng.1) zur patientenseitigen Koppeleinheit (1) zu fördern,
wobei die Ansteuer-Anordnung
- einen Temperatursensor (20, 21, 22) aus einer Temperatursensor-Gruppe und
- ein signalverarbeitendes Steuergerät (11)
umfasst,
wobei das Steuergerät (11) dazu ausgestaltet ist, eine Soll-Konzentration (con_{req}) des Narkosemittels (Nm) im zu erzeugenden Narkotisierungs-Gasgemisch (Ng, Ng.1) zu berechnen, und
wobei das Steuergerät (11) weiterhin dazu ausgestaltet ist, das Narkosemitteldosierer-Bauteil (38, 38.1) mit dem Ziel anzusteuern, dass das Narkosemitteldosierer-Bauteil (38, 38.1) das Narkotisierungs-Gasgemisch (Ng, Ng.1) mit der berechneten Soll-Konzentration (con_{req}) bereitstellt,
**dadurch gekennzeichnet, dass**
die Ansteuer-Anordnung einen Datenspeicher (33.1, 33.2) mit einem rechnerauswertbaren funktionalen Zusammenhang (32.1, 32.2) umfasst,
wobei die Temperatursensor-Gruppe aus
- einem Umgebungstemperatur-Sensor (20),
- einem narkosemitteldosierer-seitigen Temperatursensor (21) und
- einem beatmungsgeräte-seitigen Temperatursensor (22, 22.1) besteht,
wobei der Umgebungstemperatur-Sensor (20) dazu ausgestaltet ist, eine Umgebungstemperatur (Temp_{amb}) in einer Umgebung der Beatmungs-Anordnung zu messen,
wobei der narkosemitteldosierer-seitige Temperatursensor (21) dazu ausgestaltet ist, eine Temperatur (Temp₃₈) an einer Narkosemitteldosierer-Messposition im oder am Narkosemitteldosierer-Bauteil (38, 38.1) zu messen,
wobei der beatmungsgeräte-seitige Temperatursensor (22, 22.1) dazu ausgestaltet ist, eine Temperatur (Temp₅₀) an einer Beatmungsgeräte-Messposition im oder am Beatmungsgerät (100) zu messen,
wobei der funktionale Zusammenhang (32.1, 32.2) eine obere Konzentrations-Schranke (Conₘₐₓ) für die Konzentration des Narkosemittels im Narkotisierungs-Gasgemisch (Ng, Ng.1) in Abhängigkeit von einer messbaren Temperatur (Temp_{amb}, Temp₃₈, Temp₅₀) dergestalt spezifiziert,
dass die obere Konzentrations-Schranke (Conₘₐₓ) mit steigender messbarer Temperatur (Temp_{amb}, Temp₃₈, Temp₅₀) größer wird oder mindestens gleichbleibt,
wobei eine messbare Temperatur eine Temperatur ist, die von einem Temperatursensor (20, 21, 22) der Temperatursensor-Gruppe messbar ist,
wobei das Steuergerät (11) weiterhin dazu ausgestaltet ist,
- den funktionalen Zusammenhang (32.1, 32.2) auf einen gemessenen Wert (temp_{amb}, temp₃₈, temp₅₀) einer messbaren Temperatur (Temp_{amb}, Temp₃₈, Temp₅₀) anzuwenden,
- hierdurch einen Wert (conₘₐₓ) für die obere Konzentrations-Schranke (Conₘₐₓ) herzuleiten und
- die Soll-Konzentration (con_{req}) so zu berechnen, dass die Soll-Konzentration (con_{req}) höchstens gleich dem hergeleiteten Wert (conₘₐₓ) für die obere Konzentrations-Schranke (Conₘₐₓ) ist.

11. Ansteuer-Verfahren zum Ansteuern einer Beatmungs-Anordnung,
wobei die Beatmungs-Anordnung zur künstlichen Beatmung eines Patienten (Pt) ausgestaltet ist und
- ein Beatmungsgerät (100) mit einem Gehäuse (12),
- eine patientenseitige Koppeleinheit (1) und
- ein Narkosemitteldosierer-Bauteil (38, 38.1),
umfasst,
wobei die patientenseitige Koppeleinheit (1) dazu ausgestaltet ist, im und / oder am Körper eines künstlich zu beatmenden Patienten (Pt) angeordnet zu werden,
wobei das Gehäuse (12) des Beatmungsgeräts (100) eine Aufnahme (50, 50.1) umfasst,
wobei das Narkosemitteldosierer-Bauteil (38, 38.1) wenigstens zeitweise in die Aufnahme (50, 50.1) eingesetzt ist,
wobei das Narkosemitteldosierer-Bauteil (38, 38.1) dazu ausgestaltet ist, ein Narkotisierungs-Gasgemisch (Ng, Ng.1) umfassend ein Narkosemittel (Nm) zu erzeugen, und
wobei das Beatmungsgerät (100) dazu ausgestaltet ist, ein atembares Gasgemisch (Bg) umfassend Sauerstoff und das erzeugte Narkotisierungs-Gasgemisch (Ng, Ng.1) zur patientenseitigen Koppeleinheit (1) zu fördern, und
wobei das Ansteuer-Verfahren unter Verwendung
- eines Temperatursensors (20, 21, 22) und
- eines signalverarbeitenden Steuergeräts (11)
durchgeführt wird und
wobei das Ansteuer-Verfahren die Schritte umfasst, dass
- der Temperatursensor (20, 21, 22) eine messbare Temperatur (Temp_{amb}, Temp₃₈, Temp₅₀) misst,
- das Steuergerät (11) eine Soll-Konzentration (con_{req}) des Narkosemittels (Nm, Nm.1) im zu erzeugenden Narkotisierungs-Gasgemisch (Ng, Ng.1) berechnet und
- das Narkosemitteldosierer-Bauteil (38, 38.1) mit dem Ziel ansteuert, dass das Narkosemitteldosierer-Bauteil (38, 38.1) das Narkotisierungs-Gasgemisch (Ng, Ng.1) mit der berechneten Soll-Konzentration (con_{req}) bereitstellt,
**dadurch gekennzeichnet, dass**
der Temperatursensor (20, 21, 22) aus einer Temperatursensor-Gruppe ist und
das Ansteuer-Verfahren unter Verwendung eines Datenspeichers (33.1, 33.2) mit einem rechnerauswertbaren funktionalen Zusammenhang (32.1, 32.2) durchgeführt wird,
wobei die Temperatursensor-Gruppe aus
- einem Umgebungstemperatur-Sensor (20),
- einem narkosemitteldosierer-seitigen Temperatursensor (21) und
- einem beatmungsgeräte-seitigen Temperatursensor (22, 22.1) besteht,
wobei die messbare Temperatur (Temp_{amb}, Temp₃₈, Temp₅₀)
- eine Umgebungstemperatur (Temp_{amb}) in einer Umgebung der Beatmungs-Anordnung und / oder
- eine Temperatur (Temp₃₈) an einer Narkosemitteldosierer-Messposition im oder am Narkosemitteldosierer-Bauteil (38, 38.1) und / oder
- eine Temperatur (Temp₅₀) an einer Beatmungsgeräte-Messposition im oder am Beatmungsgerät (100)
ist,
wobei der funktionale Zusammenhang (32.1, 32.2) eine obere Konzentrations-Schranke (Conₘₐₓ) für die Konzentration des Narkosemittels im Narkotisierungs-Gasgemisch (Ng, Ng.1) in Abhängigkeit von einer messbaren Temperatur (Temp_{amb}, Temp₃₈, Temp₅₀) dergestalt spezifiziert,
dass die obere Konzentrations-Schranke (Conₘₐₓ) mit steigender messbarer Temperatur (Temp_{amb}, Temp₃₈, Temp₅₀) größer wird oder mindestens gleichbleibt, und
wobei der Schritt, dass das Steuergerät (11) die Soll-Konzentration (con_{req}) berechnet, die Schritte umfasst, dass das Steuergerät (11)
- den funktionalen Zusammenhang (32.1, 32.2) auf einen gemessenen Wert (temp_{amb}, temp₃₈, temp₅₀) einer messbaren Temperatur (Temp_{amb}, Temp₃₈, Temp₅₀) anwendet,
- hierdurch einen Wert (conₘₐₓ) für die obere Konzentrations-Schranke (Conₘₐₓ) herleitet und
- die Soll-Konzentration (con_{req}) so berechnet, dass die Soll-Konzentration (con_{req}) höchstens gleich dem hergeleiteten Wert (conₘₐₓ) für die obere Konzentrations-Schranke (Conₘₐₓ) ist.
